# EUROPEAN PATENT APPLICATION

(11) **EP 4 360 643 A1**
(43) Date of publication of application: **01.05.2024**
(21) Application number: 22383040.7
(22) Date of filing: 28.10.2022
(51) Int. Cl.: A61K 38/17, A61K 38/12, A61K 38/04, A61P 1/16

(54) **SOCS1 DERIVED PEPTIDES FOR USE IN THE TREATMENT OF LIVER DISEASES**

(71) Applicant: Fundacion Instituto De Investigacion Sanitaria Fundacion Jimenez Diaz, 28040 Madrid (ES); Universidad Autónoma de Madrid, 28049 Madrid (ES)
(72) Inventor: EGIDO DE LOS RÍOS, Jesús, 28040 Madrid (ES); GÓMEZ GUERRERO, Carmen, 28040 Madrid (ES); MAS FONTAO, Sebastián, 28040 Madrid (ES); SOTO CATALÁN, Manuel, 28040 Madrid (ES); MARASCO, Daniela, 28040 Madrid (ES); LA MANNA, Sara, 28040 Madrid (ES); BERNAL URIBE, Susana, 28040 Madrid (ES); OPAZO RÍOS, Lucas, 28040 Madrid (ES); PRIETO ARROYO, Ignacio, 28040 Madrid (ES); LÓPEZ SANZ, Laura, 28040 Madrid (ES); JIMÉNEZ CASTILLA, Luna, 28040 Madrid (ES)
(74) Representative: Hoffmann Eitle

(57) **Abstract**

The invention relates to SOCS1 derived isolated polypeptides and compositions comprising such polypeptides for use in the prevention, relief and/or treatment of an overweight/obesity and diabetes related liver disorder or a pathological condition or disease caused by fatty liver, specifically non-alcoholic fatty liver disease (NAFLD) or non-alcoholic steatohepatitis (NASH) or metabolic associated fatty liver disease (MAFLD).

## Description

### Technical field

The invention relates to SOCS1 derived isolated polypeptides and compositions comprising such polypeptides for use in the prevention, relief and/or treatment of an obesity related or an overweight related liver disorder or a pathological condition or disease caused by fatty liver, specifically non-alcoholic fatty liver disease (NAFLD).

### Background of the invention

Parallel to the increasing prevalence of obesity and diabetes and close related with insulin resistance and metabolic risk factors, non-alcoholic fatty liver disease (NAFLD) has become the most common chronic liver disease in the world over the past 30 years. The prevalence of NAFLD ranges from 17% to 46% and it is rising in recent years due to changes in dietary habits and increase of sedentary lifestyle. In high-risk groups of severe obesity, diabetes and dyslipidemia, the prevalence of NAFLD was found to be 90%, 69% and 50%, respectively [1]. It is estimated that the prevalence of diagnosed NAFLD will reach 18 million by 2027 in US, Japan, and EU 5 (England, France, Germany, Italy, and Spain) [2, 3].

NAFLD can range from simple steatosis to non-alcoholic steatohepatitis (NASH), characterized by liver inflammation and hepatocyte ballooning, with or without fibrosis [4]. NAFLD patients have a 1.7-fold increase in standardized age and gender-matched mortality. The most common cause of death in patients with NAFLD/NASH is cardiovascular events, but they also have an increased liver-related mortality rate particularly when advanced fibrosis, cirrhosis, and hepatocellular carcinoma develop [5]. For these advanced patients, liver transplantation is the only therapy currently available. Indeed, NAFLD/NASH is likely to become the leading cause for liver transplantation over the next 20 years. Given the high prevalence and economic burden of NAFLD ($100 billion in annual direct medical costs in US) [2] and the fact that no treatment is available, a better understanding of its pathogenesis is essential to identify targets for drug development to overcome the epidemic.

The steatosis stage is characterized by triglyceride accumulation in hepatocytes through three possible mechanisms: 1. Fatty acid uptake from the bloodstream into the hepatocyte via increased transmembrane receptors such as cluster of differentiation 36 (CD36), also known as fatty acid translocase (FAT), fatty acid transport protein 2 (FATP2), fatty acid transport protein 5 (FATP5), among others. 2. Failure to scavenge lipids from within the hepatocyte mainly due to reduced reverse transport. 3. Increased synthesis of new fatty acids, a process known as de novo lipogenesis. The onset of NAFLD/NASH is associated with multiple cellular stresses in lipid-loaded hepatocytes, including mitochondrial damage, endoplasmic reticulum stress, oxidative stress and/or lipotoxicity. Liver cellular stress involves the accumulation of saturated fatty acids, increased de novo lipogenesis resulting from increased fructose uptake, or cholesterol accumulation in the endoplasmic reticulum. In addition to increasing de novo lipogenesis, fructose also increases intestinal permeability, which can lead to the activation of hepatic immune cells by gut-derived inflammatory mediators. This promotes liver inflammation and the death of stressed hepatocytes, which also release pro-inflammatory mediators such as ATP, extracellular vesicles, chemokines and cytokines, further reinforcing the inflammatory process and fuelling the transition from steatosis to NASH. Exacerbation of this vicious cycle ultimately leads to cirrhosis and eventually hepatocellular carcinoma [6-8].

Nowadays, there is no specific medical therapy for NAFLD. Lifestyle modifications are the main intervention for NASH without fibrosis. Adjunctive treatment with vitamin E or pioglitazone, an insulin-sensitizing agent with anti-inflammatory actions, might also be considered. Several large clinical trials designed to identify effective and safe treatments for NASH and liver fibrosis are in progress [9]. Efforts have focused on ameliorating the three key processes in the pathogenesis and progression of NASH: metabolic stress, inflammation, and fibrosis. Agents targeting metabolism include inhibitors of de novo fatty acid synthesis, stearoyl-CoA desaturase 1 inhibitors, diacylglycerol acyltransferase inhibitors, mitochondrial pyruvate carrier inhibitors, farnesoid X receptor agonists and PPAR Dual Agonists. Although results have been reported for several studies, no agent has yet received approval by the Food and Drug Administration for the treatment of NASH. Novel investigational drugs aimed to reduce inflammation and/or fibrosis (i.e. CCR2/CCR5 inhibitors, anti-TNFa, galectin-3 inhibitor) are still on early development phases [10].

The mechanisms contributing to the NAFLD/NASH development and the progression to hepatocarcinoma even in the absence of NASH and/or overt fibrosis or cirrhosis have remained unexplored. Among them, the Janus kinase/signal transducers and activators of transcription (JAK/STAT) signaling pathway is a central mechanism for a variety of metabolically relevant cytokines, growth factors, hormones, and lipids [11]. Dysregulated JAK/STAT activation has been associated to human progressive diabetic kidney disease, cardiovascular diseases, and metabolic disorders including NAFLD [12, 13]. In NAFLD/NASH patients, the oxidative hepatic environment in obesity increases STAT1 and STAT3 signaling associated to cytokines. In obese mouse models, protein tyrosine phosphatase-mediated STAT1 inactivation prevented T cell recruitment, NASH and fibrosis, while STAT3 inactivation prevents hepatocellular carcinoma [14, 15]. Hepatocyte-specific deletion of JAK2 prevents steatohepatitis and hepatocarcinogenesis in a NAFLD dietary model [16].

The protein family of Suppressors of cytokine signaling (SOCS) is composed of 8 members (CIS, and SOCS1-7) that function as endogenous negative regulators to control the magnitude and duration of JAK/STAT signaling pathway. Several SOCS members have been suggested as potential therapeutic target to prevent pathological activation of JAK/STAT in different pathologies including cardiovascular, inflammatory and renal diseases [17-19]. The inventors have previously characterized several peptidomimetics of SOCS1 and SOCS3 proteins with promising antioxidant and anti-inflammatory properties [20-22]. Nevertheless, the benefit of SOCS-based therapy in liver metabolic complications remains to be studied.

Despite the research existing in this field and the fact that the relationship between the JAK/STAT signaling pathway and SOCS proteins has been postulated, the effective administration of a peptide per se for the prevention or treatment of an obesity related liver disorder or a pathological condition or disease caused by fatty liver has not been described up until now. SOCS mimetic peptides have not been linked in any case to such disorders.

There is therefore a need for the development of improved treatment options for obesity related liver disorders or pathological conditions or diseases caused by fatty liver, such as specifically NAFLD/NASH.

### Summary of the Invention

The present invention therefore relates to isolated polypeptides for use in the prevention, relief and/or treatment of an obesity related or overweight related liver disorder or a pathological condition or disease caused by fatty liver.

In one embodiment the present invention relates to an isolated polypeptide comprising
a) the sequence of SEQ ID NO.1 or SEQ ID NO.2; or
b) a variant of any of the sequences of a) which is at least 80%, 81%, 82%, 83%, 84%, 85%, 86%, 87%, 88%, 89%, 90%, 91%, 92%, 93%, 94%, 95%, 96%, 97%, 98%, 99% identical SEQ ID NO.1 or SEQ ID NO.2, respectively,
for use in the prevention, relief and/or treatment of an overweight related or obesity related liver disorder or a pathological condition or disease caused by fatty liver, specifically non-alcoholic fatty liver disease (NAFLD) or non-alcoholic steatohepatitis (NASH) or metabolic associated fatty liver disease (MAFLD).

In a further embodiment the isolated polypeptide further comprises at least one of the following amino acid substitutions based on the amino acid sequence of SEQ ID NO.1: H3C(Acm), F4R, R5Q, F7Nal1, S9K, A11S, a deletion of amino acids A11 to 116, or a combination thereof.

In one embodiment the polypeptide comprises
a) the sequence of SEQ IN NO.3 or SEQ ID NO.4; or
b) a variant of any of the sequences of a) which is at least 85%, 86%, 87%, 88%, 89%, 90%, 91%, 92%, 93%, 94%, 95%, 96%, 97%, 98%, 99% identical to SEQ ID NO.2, SEQ IN NO.3 or SEQ ID NO.4, respectively.

In one preferred embodiment the pathological condition or disease is an obesity related liver disorder selected from steatosis, steatohepatitis, liver fibrosis or cirrhosis, non-alcoholic steatohepatitis (NASH), non-alcoholic fatty liver disease (NAFLD) and NAFLD related cirrhosis.

In a preferred embodiment the polypeptide consists of
i. the amino acid sequence of SEQ ID NO.1; or
ii. the amino acid sequence of SEQ ID NO.2; or
iii. the amino acid sequence of SEQ ID NO.3; or
iv. the amino acid sequence of SEQ ID NO.4.

In a further preferred embodiment, the polypeptide consists of
a) the murine SOCS1 protein (UniProt: 035716);
b) the human SOCS1 protein (UniProt: 015524); or
c) a variant of the sequence of a) or b) which is at least 80% homologous to the amino acid sequence of the murine SOCS1 protein or to the amino acid sequence of the human SOCS1 protein.

In one embodiment the polypeptide consists of any one of SEQ ID NO.1, SEQ ID NO.2, SEQ ID NO.3 or SEQ ID NO.4, wherein the polypeptide is bound to a cell permeability region.

In a further embodiment of the polypeptide of present invention at least one amino acid of the sequence thereof is phosphorylated, preferably wherein at least one of the phosphorylated amino acids is a tyrosine (Y). In a preferred embodiment the polypeptide is SEQ ID NO.1 or derivatives thereof.

In a further aspect present invention relates to a composition, preferably a pharmaceutical composition, comprising a therapeutically effective amount of a polypeptide as defined herein and at least one pharmaceutically acceptable vehicle or excipient, for use in the prevention, relief and/or treatment of an obesity related liver disorder or a pathological condition or disease caused by fatty liver, preferably wherein the obesity related liver disorder or pathological condition or disease caused by fatty liver is selected from steatosis, steatohepatitis, liver fibrosis or cirrhosis, non-alcoholic steatohepatitis (NASH), non-alcoholic fatty liver disease (NAFLD) and NAFLD related cirrhosis.

In one embodiment the pharmaceutical composition further comprises at least one other active ingredient.

The present in invention also relates to a pharmaceutical composition comprising the peptides as described herein further comprising pharmaceutically acceptable excipients.

In one embodiment the composition is suitable for oral, gastroenteric, parenteral, or rectal administration, preferably parenteral administration.

### BRIEF DESCRIPTION OF THE FIGURES

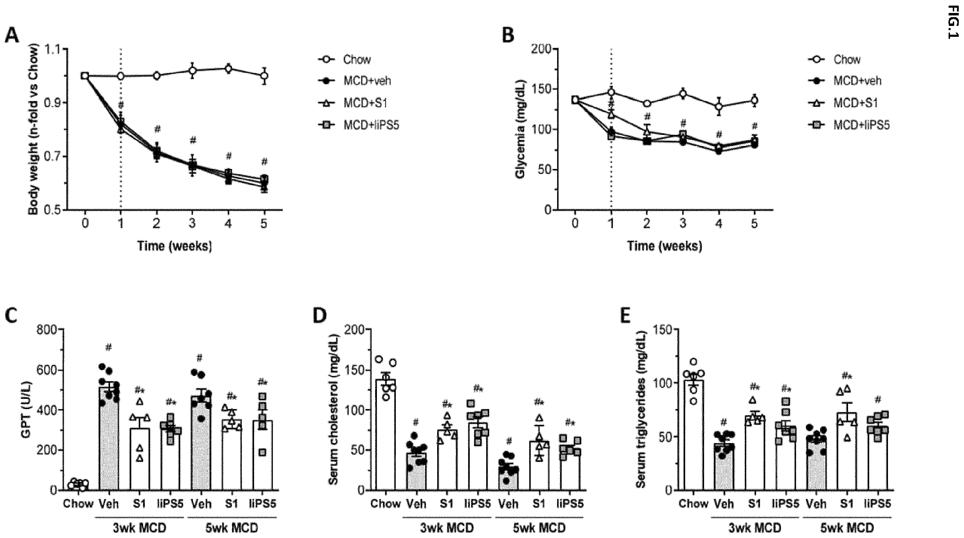

Figure 1: Characterization of the experimental model of NAFLD/NASH induced by MCD diet in mice. Evolution of body weight (A) and non-fasting blood glucose levels (B) in WT mice were fed with MCD diet for 3 and 5 weeks and treated with vehicle (Veh) or peptides (S1 and liPS5). Vertical dashed lines at 1 week indicate the initiation of treatment. A group of mice on Chow diet served as control. Serum levels of GPT transaminase (C), total cholesterol (D) and triglycerides (E) in the experimental groups at 3 and 5 weeks. Results are presented as individual data points and mean ± SEM of total number of animals per group. #p<0.05 vs Chow and *p<0.05 vs MCD.

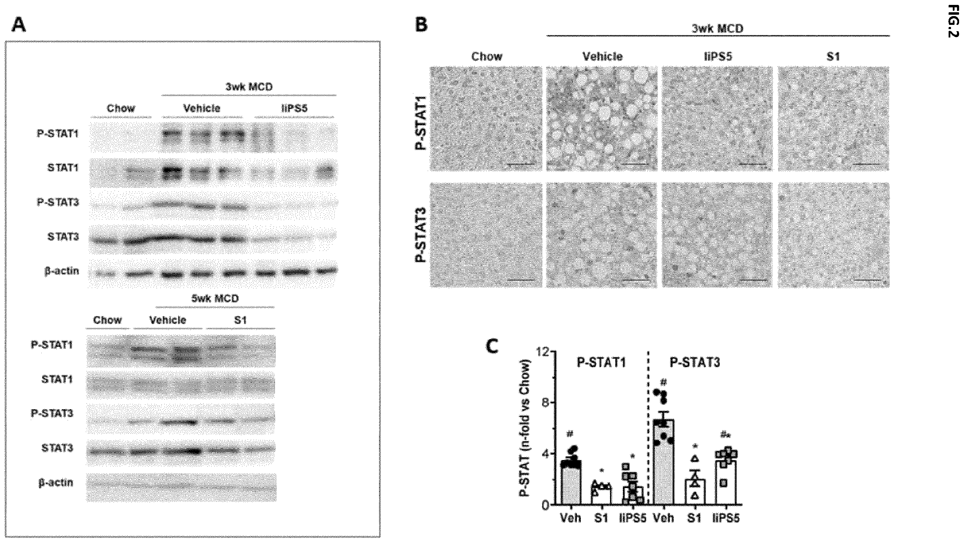

Figure 2: Inhibition of JAK/STAT pathway activation in experimental NAFLD model induced by MCD diet. (A) Western blot analysis of total and phosphorylated (P-) STAT1 and STAT3 (β-actin as loading control) in liver samples of WT mice were fed with Chow diet and MCD diet treated with vehicle (Veh) and peptides (S1 and liPS5) for 3 and 5 weeks. (B) Representative images (scale bars, 50 µm) of the immunohistochemical detection of P-STAT1 and P-STAT3 in mouse liver sections at 3 weeks of study. (C) Quantification of positive stained area in the experimental groups. Results are presented as individual data points and mean±SEM of total number of animals per group. #p<0.05 vs Chow and *p<0.05 vs MCD.

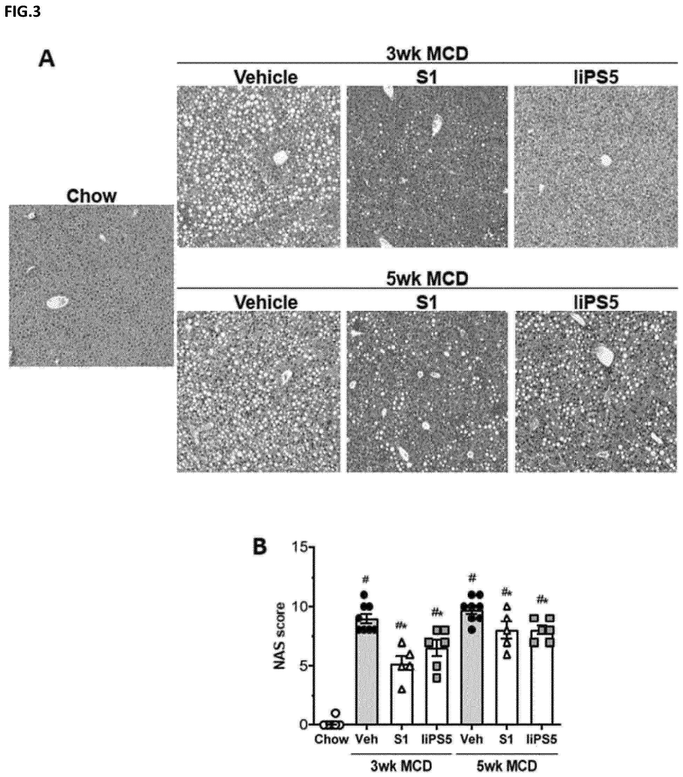

Figure 3: SOCS1-derived peptides alleviate liver damage induced by MCD diet in mice. (A) Representative images (scale bars, 50 µm) of hematoxylin/eosin staining in mouse liver sections after feeding with MCD for 3 and 5 weeks (treatment with vehicle (Veh), S1 and liPS5 peptides), compared with control group (Chow diet). (B) Total NAS score calculated from individual scores for steatosis, lobular inflammation and ballooning. Results are presented as individual data points and mean±SEM of total number of animals per group. #p<0.05 vs Chow and *p<0.05 vs MCD.

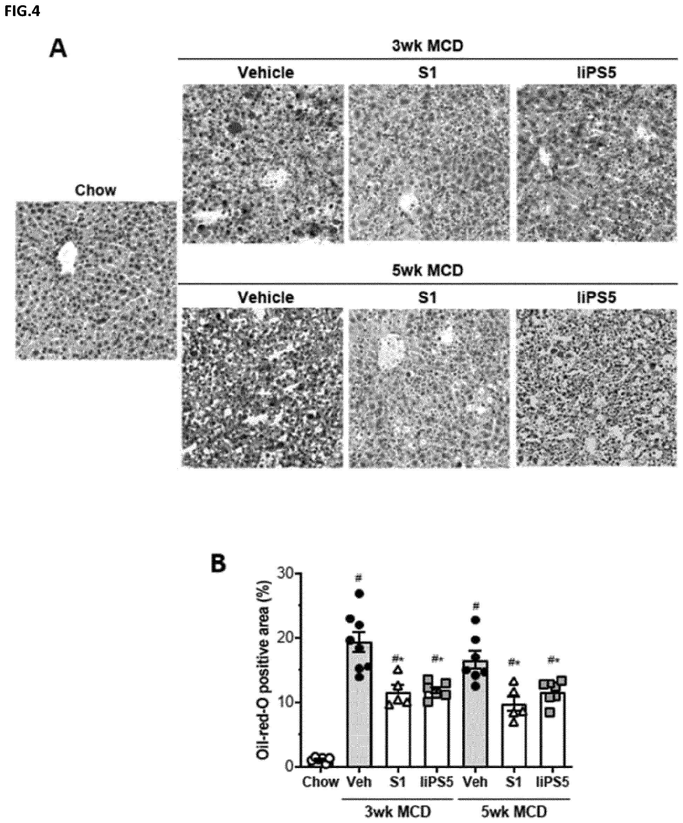

Figure 4: SOCS1-derived peptides reduce hepatic steatosis in MCD diet fed mice. (A) Representative images (scale bars, 50 µm) of Oil red O/hematoxylin staining in mouse liver sections after feeding with MCD for 3 and 5 weeks (vehicle, S1 and liPS5 groups), compared with control group (Chow diet). (B) Quantification of the percentage of Oil red O-stained area, as marker of fat accumulation in the liver. Results are presented as individual data points and mean±SEM of total number of animals per group. #p<0.05 vs Chow and *p<0.05 vs MCD.

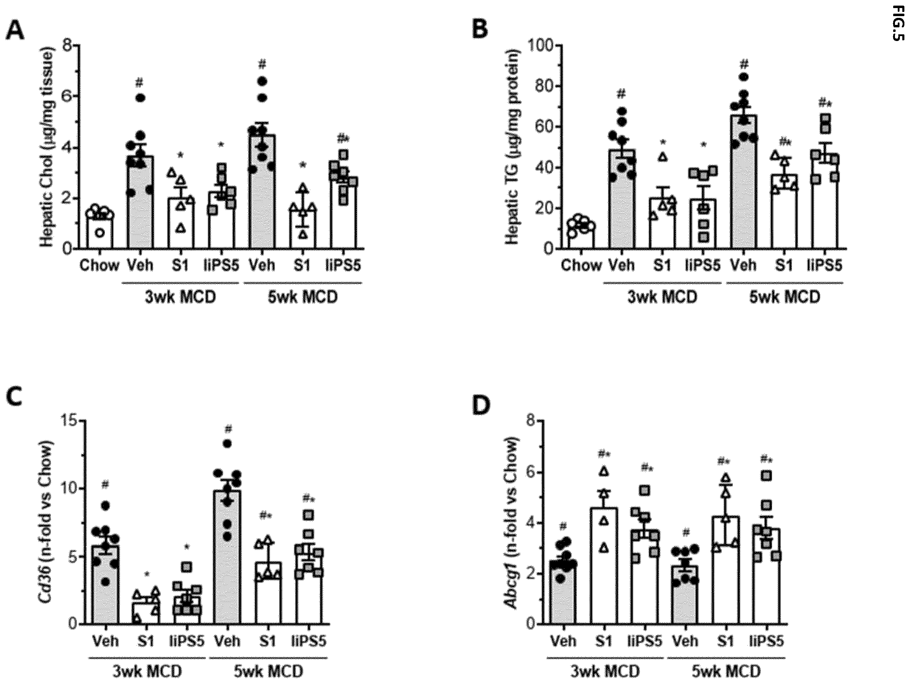

Figure 5: Effect of S1 and liPS5 peptides on hepatic lipid accumulation in MCD-fed mice. (A) Hepatic total cholesterol content expressed in micrograms per gram of liver weight. (B) Liver triglyceride content expressed per mg of protein content. (C-D) Real-time PCR analysis of fatty acid uptake- and eflux-related gene expression in mouse liver samples. Values normalized to 18S rRNA are expressed as fold increases versus Chow group. Results are presented as individual data points and mean±SEM of total number of animals per group. #p<0.05 vs Chow and *p<0.05 vs MCD.

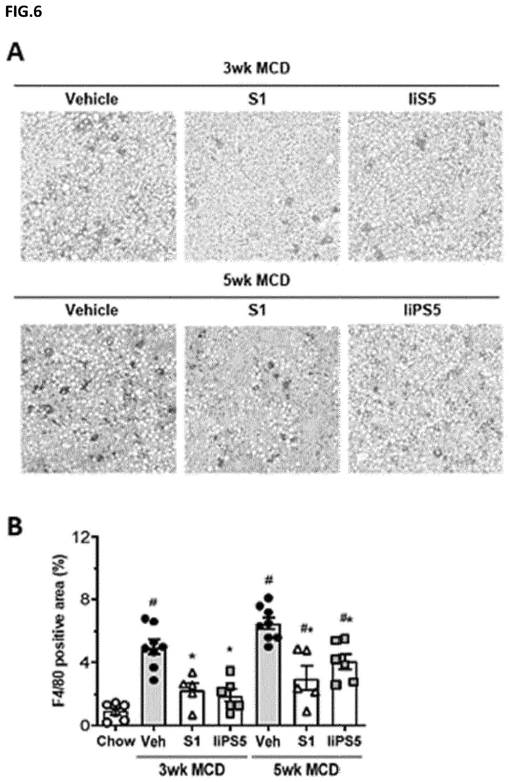

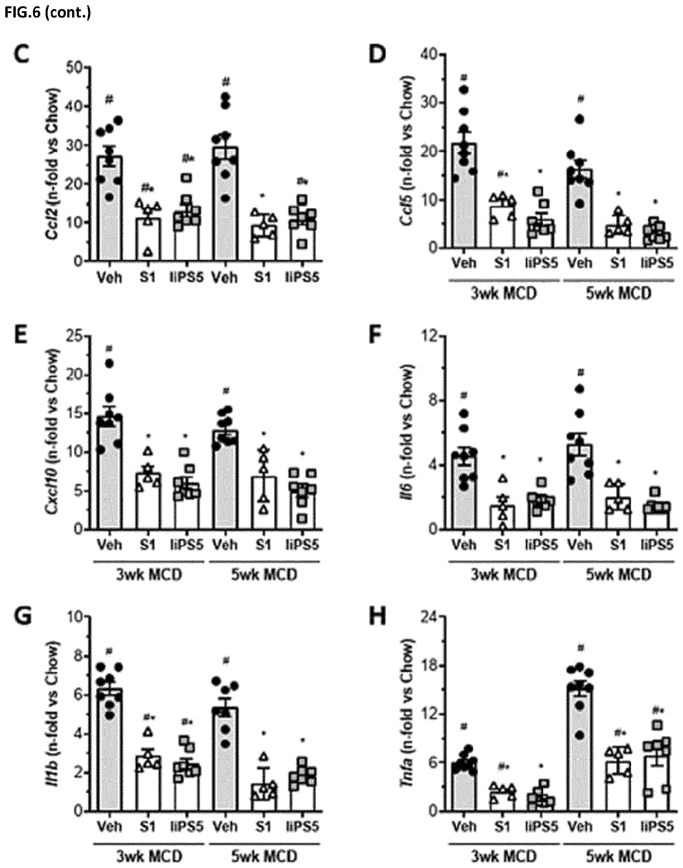

Figure 6: SOCS1-derived peptides alleviate hepatic inflammation in MCD diet fed mice. Representative images (scale bars, 50 µm) (A) and quantification of positive stained area (B) of F4/80 immunohistochemistry, as marker of macrophages and liver inflammation. (C-H) Real-time PCR analysis of the gene expression of chemokines (Ccl2, Ccl5 and Cxcl10) and cytokines (I¡LI6, IL1b and Tnfa) in mouse liver samples. Values normalized to 18S rRNA are expressed as fold increases versus Chow group. Results are presented as individual data points and mean±SEM of total number of animals per group. #p<0.05 vs Chow and *p<0.05 vs MCD.

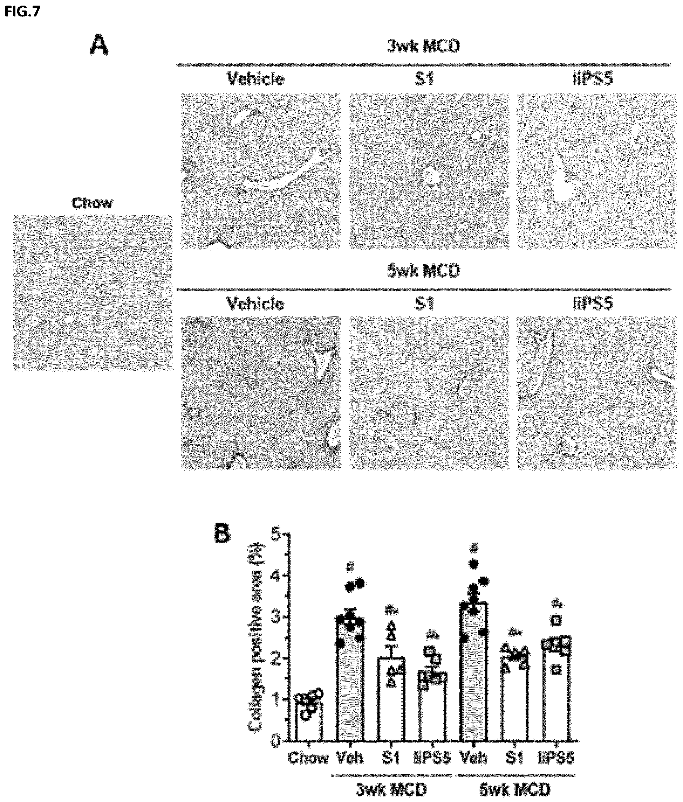

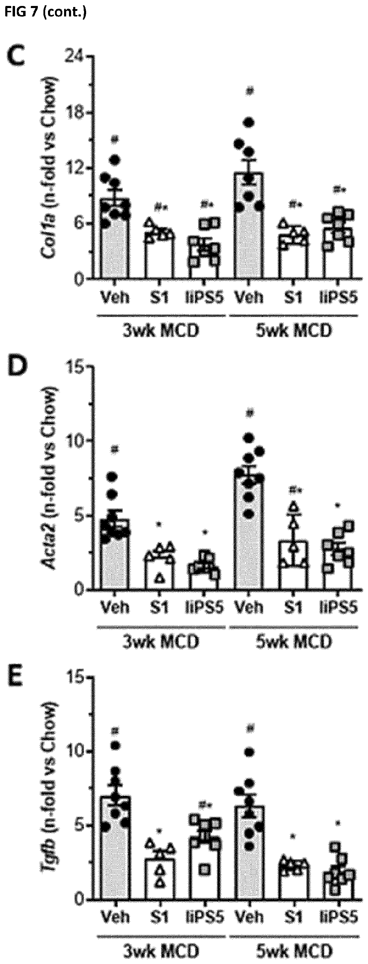

Figure 7: Effect of S1 and liPS5 peptides on hepatic fibrosis in MCD-fed mice. Representative images (scale bars, 50 µm) (A) and quantification of positive stained area (B) of Sirius red staining in mouse liver sections. (C-E) Real-time PCR analysis of profibrotic genes in mouse liver samples. Values normalized to 18S rRNA are expressed as fold increases versus Chow group. Results are presented as individual data points and mean±SEM of total number of animals per group. #p<0.05 vs Chow and *p<0.05 vs MCD.

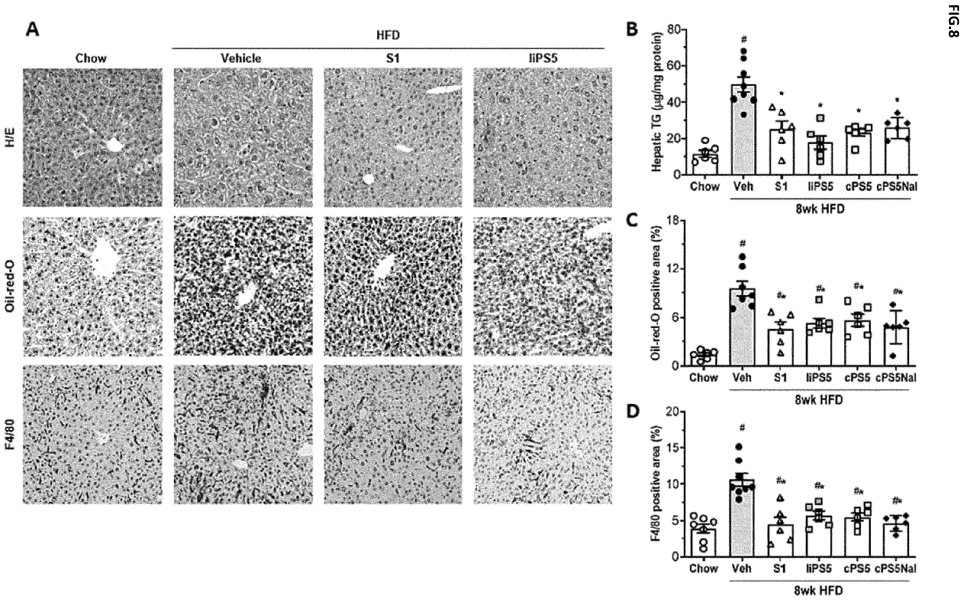

Figure 8: SOCS1-derived peptides prevent the development of NAFLD induced by high-fat diet. ApoE^{-/-} mice were fed with HFD and treated with vehicle (Veh) or S1 peptide and its derivatives (liPS5, cPS5 and cPS5Nal) for 8 weeks. A group of mice on Chow diet served as control. (A) Representative images (scale bars, 50 µm) of the histological evaluation of liver damage (hematoxylin/eosin staining, H/E), steatosis (Oil red O staining) and macrophage content (F4/80 immunohistochemistry) in mouse liver sections. (B) Liver triglyceride content expressed per mg of protein content. (C) Quantification of the percentage of Oil red O-stained area. (D) Quantification of F4/80 positive stained area. Results are presented as individual data points and mean ± SEM of total number of animals per group. #p<0.05 vs Chow and *p<0.05 vs MCD.

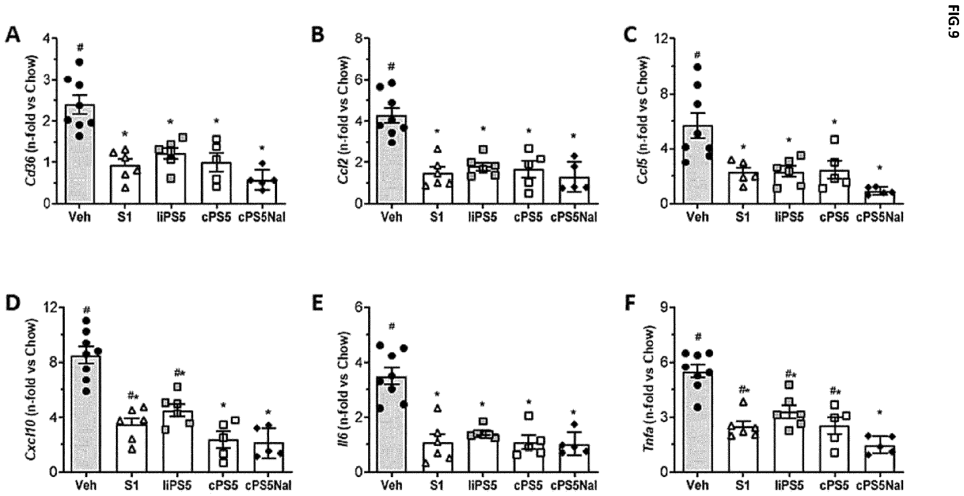

Figure 9: SOCS1-derived peptides reduced the expression of genes involved in lipid transport and inflammation in HFD in mice. Real-time PCR analysis of the mRNA expression of scavenger receptor (A) chemokines (B-D) and cytokines (E-F) in mouse liver samples. Values normalized to 18S rRNA are expressed as fold increases versus Chow group. Results are presented as individual data points and mean ± SEM of total number of animals per group. #p<0.05 vs Chow and *p<0.05 vs MCD.

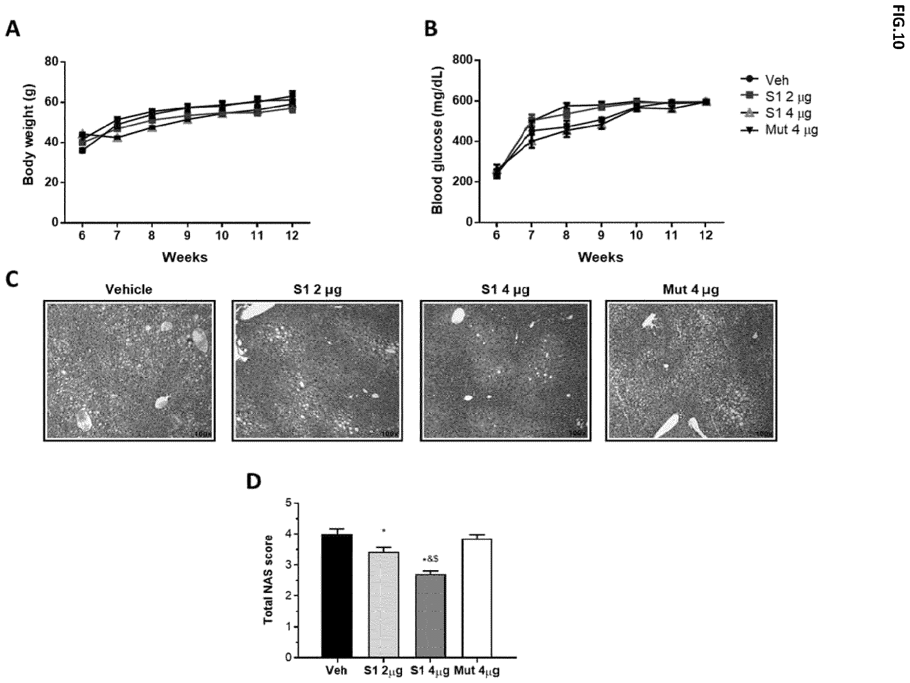

Figure 10: Effect of S1 peptide on early NAFLD development in the obesity and T2D mouse model. Evolution of body weight (A) and non-fasting blood glucose levels (B) in BTBR ob/ob mice (6 weeks old) treated with vehicle (Veh), two doses of S1 peptide (2 and 4 µg/g) and inactive mutant peptide (Mut, 4 µg/g) for 7 weeks. (C) Representative images (magnification 100x) of hematoxylin/eosin staining in mouse liver sections. (D) Total NAS score calculated from individual scores for steatosis, lobular inflammation and ballooning. Results are presented as mean±SEM of total number of animals per group. *p<0.05 vs Veh, &p<0.05 vs Mut and $p<0.05 versus S1 2 µg.

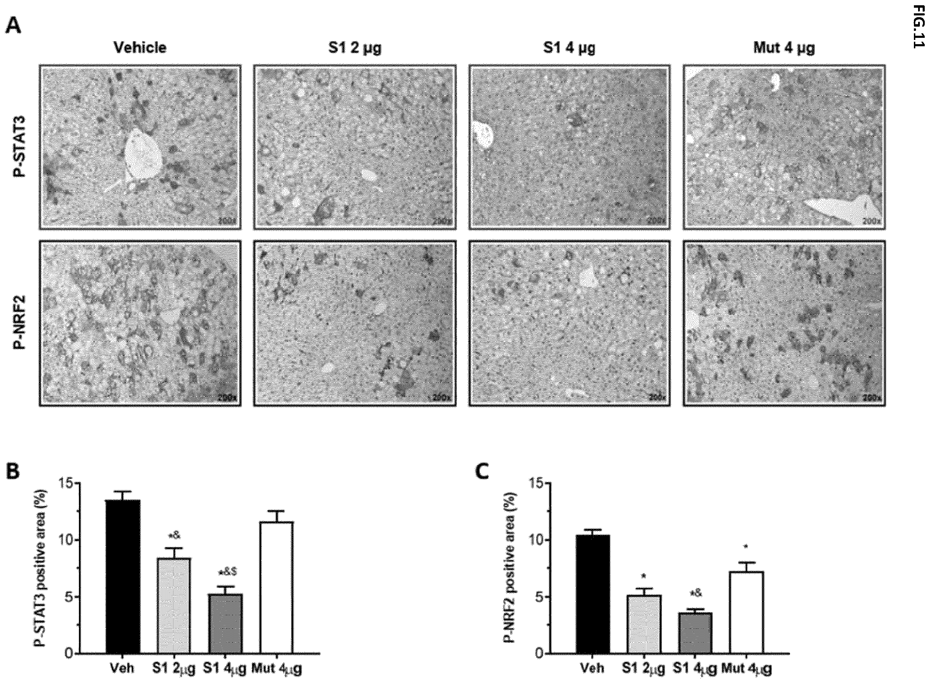

Figure 11: S1 peptide prevents early activation of STAT and NRF2 pathways in the liver of mice with obesity and T2D. (A) Representative images (magnification 200x) of the immunohistochemical detection of phosphorylated-STAT3 (P-STAT3) and phosphorylated-NRF2 (P-NRF2) in liver sections from BTBR ob/ob mice treated with vehicle, S1 and Mut peptides. Quantification of positive stained area of P-STAT3 (B) and P-NRF2 (C) in the experimental groups. Results are presented as mean ± SEM of total number of animals per group. *p<0.05 vs Veh, &p<0.05 vs Mut and $p<0.05 versus S1 2 µg.

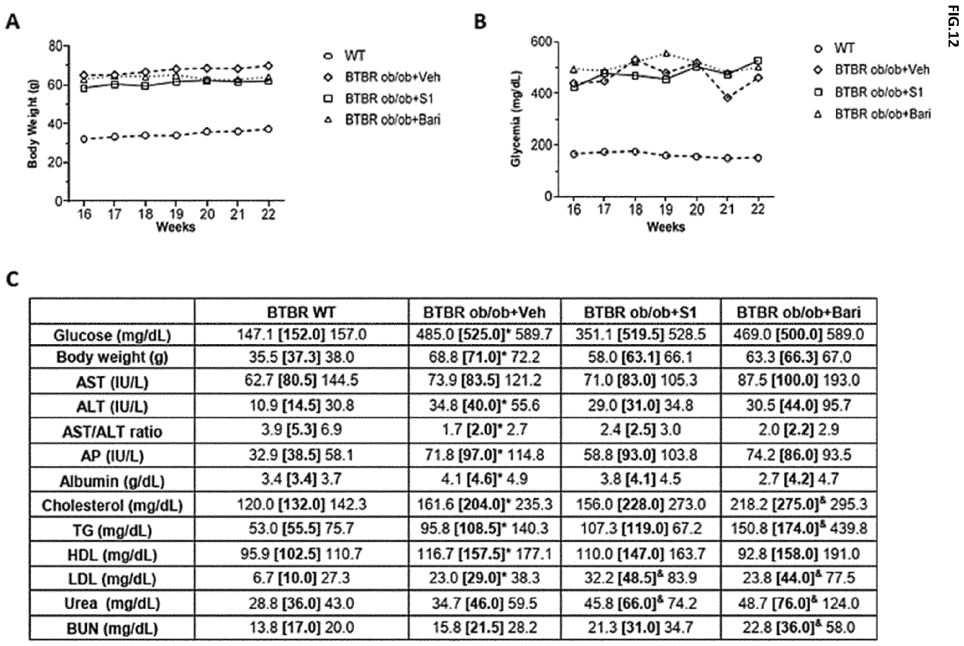

Figure 12: Effect of JAK/STAT inhibition on NAFLD progression in the obesity and T2D mouse model. Evolution of body weight (A) and non-fasting blood glucose levels (B) in BTBR ob/ob mice (16 weeks old) treated with vehicle (Veh), S1 peptide and JAK1/2 inhibitor (Bari) for 7 weeks. (C) Summary of the metabolic and biochemical parameters at the end of the study in the experimental groups (22 weeks). Results are presented as mean ± SEM of total number of animals per group. *p<0.05 versus WT and &p<0.05 versus Veh.

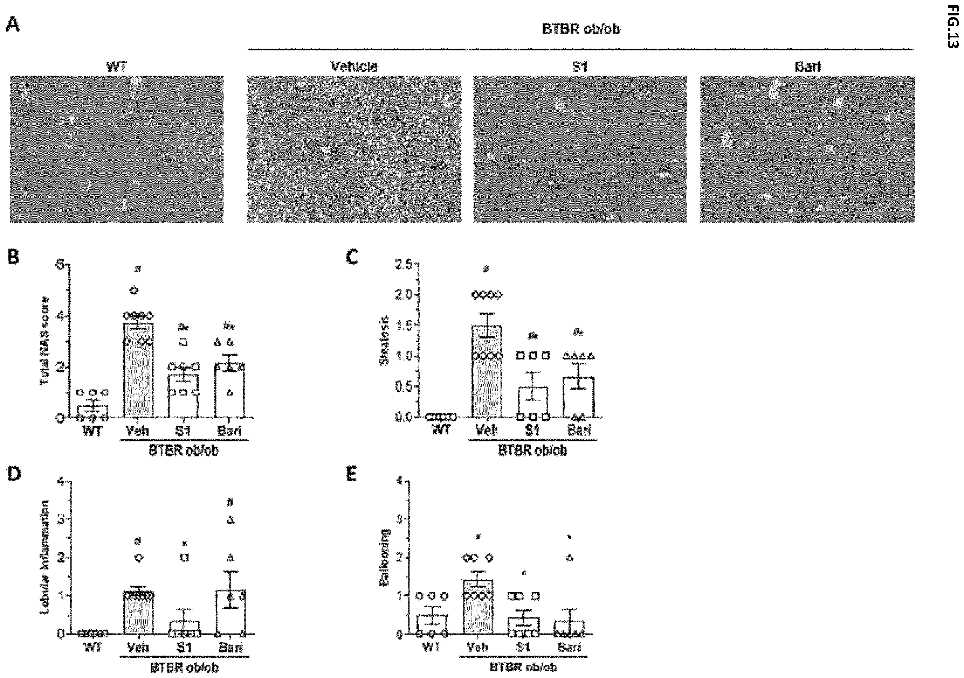

Figure 13: JAK/STAT inhibition alleviate liver damage induced by obesity and T2D in BTBR ob/ob (leptin-deficient) mice. (A) Representative images of hematoxylin/eosin staining in liver sections from WT and BTBR ob/ob mice (Veh, S1 and Bari groups) at 22 weeks. Assessment of total NAS score (B), steatosis (C), lobular inflammation (D) and ballooning (E) in the experimental groups. Results are presented as individual data points and mean ± SEM of total number of animals per group. #p<0.05 vs WT and *p<0.05 vs BTBR ob/ob.

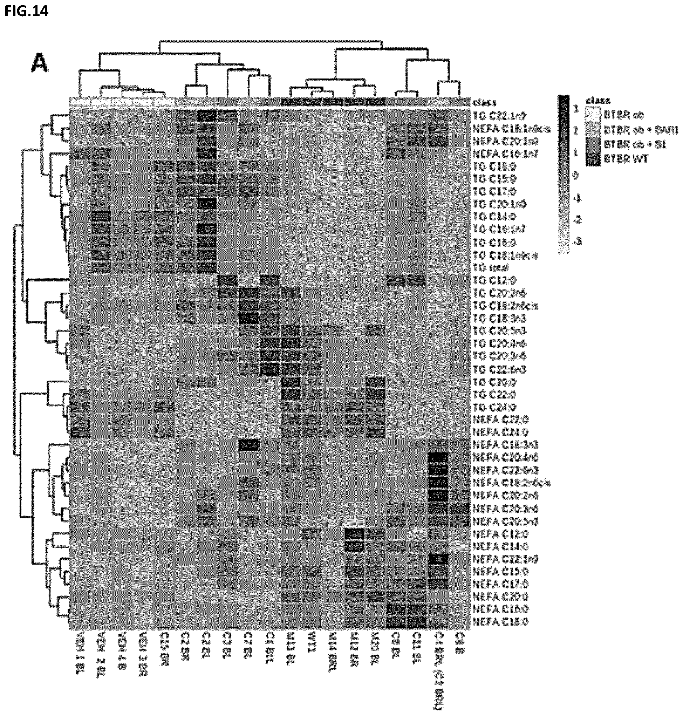

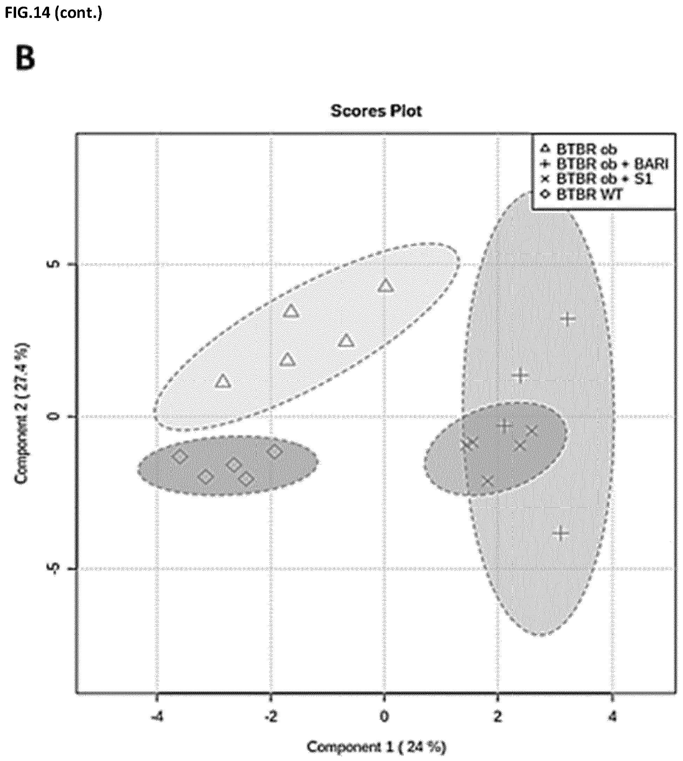

Figure 14: Lipid profile in liver samples from mice with obesity and T2D. (A) Hierarchical clustering (Pearson) based on intrahepatic lipid composition data in WT and BTBR ob/ob (untreated and treated with S1 or Bari) showing the upregulated lipids (dark brown) and downregulated (dark blue) in each experimental group. Although the effect is heterogeneous, many animals are classified together with WT and in all cases changes in lipid composition are observed. (B) Orthogonal partial least squares discriminant analysis. Component 1 segregates treated animals from untreated animals, component 2 segregates ob/ob from WT mice, while treated animals show intermediate characteristics.

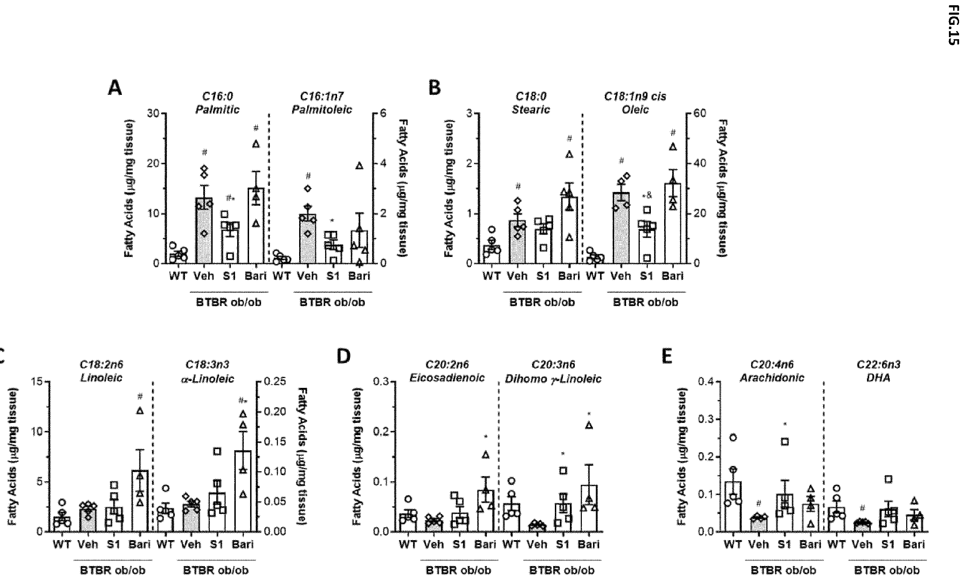

Figure 15: Analysis of lipid composition in liver at 22 weeks in BTBR ob/ob mice. Levels of de novo lipogenesis markers (A-B) and polyunsaturated fatty acids (PUFA) (C-E) were evaluated by lipidomic analysis from liver triglyceride subfraction of WT and BTBR ob/ob mice (Veh, S1 and Bari). Results are presented as individual data points and mean ± SEM of total number of animals per group. #p<0.05 versus WT, *p<0.05 versus Veh, and $p<0.05 versus Bari. DHA, docosahexaenoic acid.

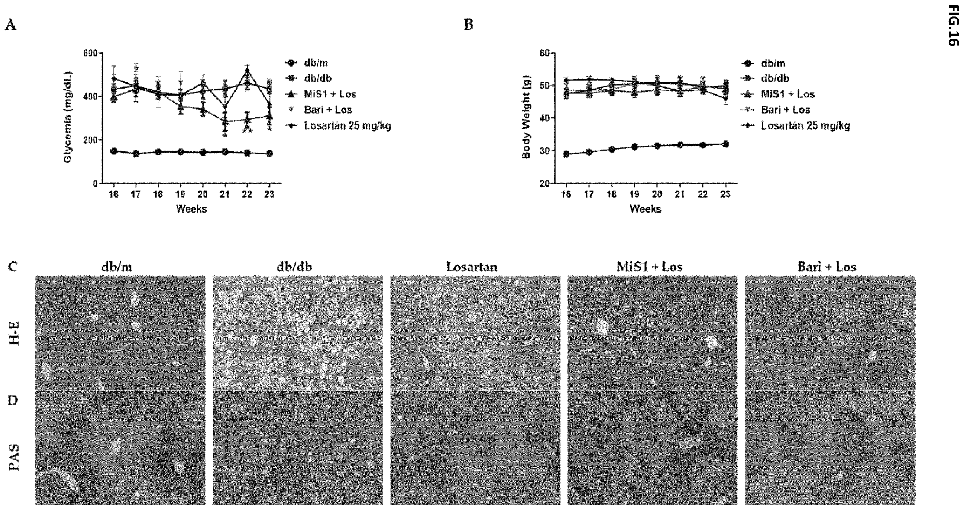

Figure 16: Hepatoprotective effects of JAK/STAT inhibition plus angiotensin receptor blocker (Losartan) alleviate obesity and T2D-induced liver damage in BKS db/db (leptin receptor deficient) mice. Evolution of body weight (A) and non-fasting blood glucose levels (B) in non-diabetic db/m mice (16 weeks-old), untreated diabetic mice (db/db) and db/db mice treated with Losartan, S1 peptide and JAK1/2 inhibitor (Bari) for 7 weeks. Results are presented as individual data points and mean ± SEM of total number of animals per group. #p<0.05 vs db/m and *p<0.05 vs db/db. (C-D) Representative images of hematoxylin/eosin staining (C) and Periodic Acid Schiff staining (D) in liver sections from non-diabetic mice (db/m), untreated diabetic mice (db/db), db/db mice treated with Losartan, S1 and Bari groups at 23 weeks.

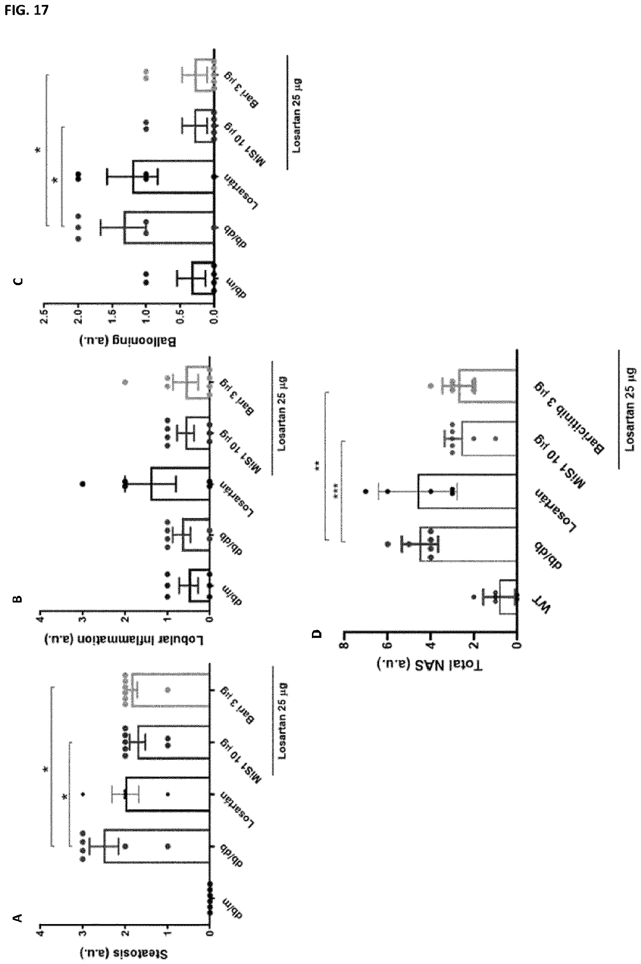

Figure 17: Assessment of steatosis (A), lobular inflammation (B), ballooning (C) and total NAS score (D) in the experimental groups of BKS db/db mouse model at 23 weeks. Results are presented as individual data points and mean ± SEM of total number of animals per group. *p<0.05, **p<0.01 and ***p<0.001 vs db/db.

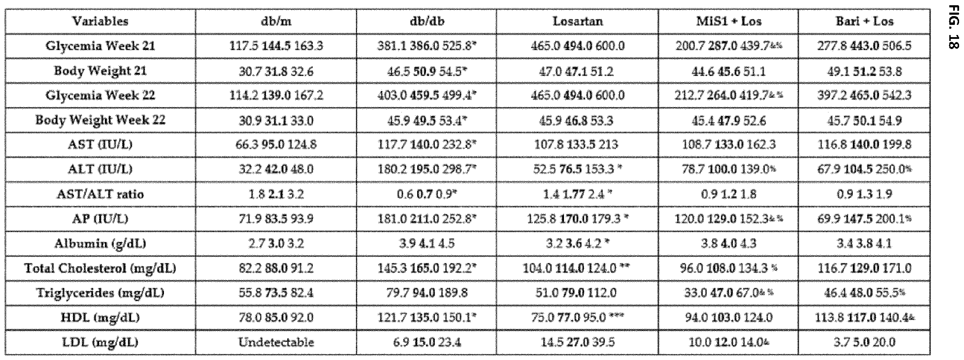

Figure 18: Summary of the metabolic and biochemical parameters at the end of the study in the experimental groups of BKS db/db mouse model at 23 weeks. Results are presented as median ± interquartile range of total number of animals per group. *p<0.05 versus db/m; &p<0.05 versus losartan and % p<0.05 versus db/db group.

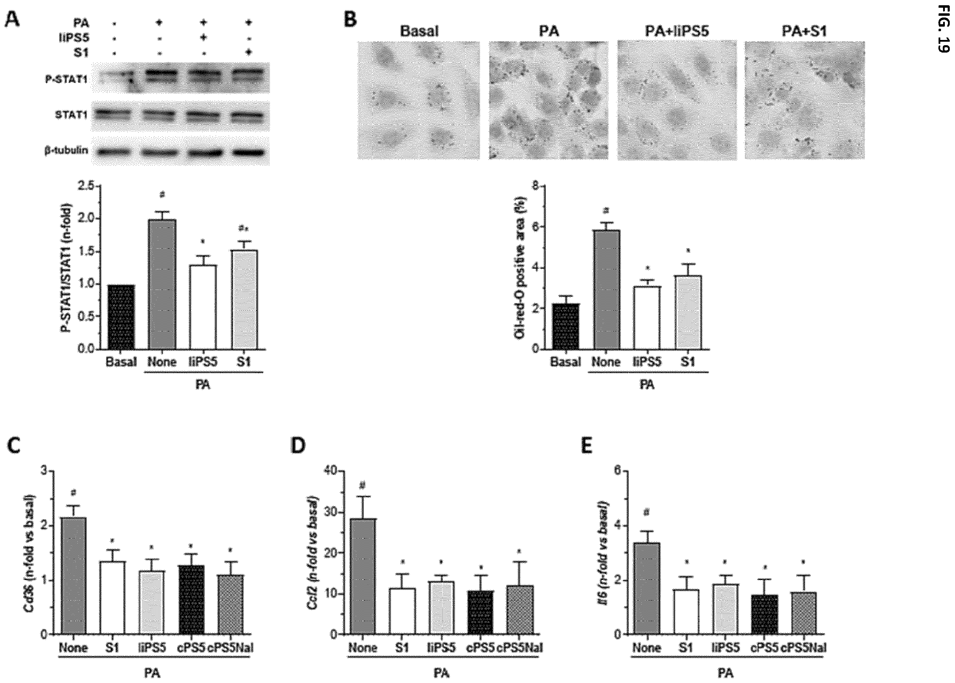

Figure 19: In vitro effects of SOCS1-derived peptides in hepatocytes. (A) Western blot analysis of P-STAT1, STAT1 and β-tubulin (loading control) in hepatocytes upon 6 hours of exposure to palmitic acid (PA) in the absence/presence of S1 and liPS5 peptides. Representative blots and summary of normalized quantification are shown. (B) Accumulation of intracellular lipids in hepatocytes at 2 hours. Representative images of Oil red O staining and quantification of positive area shown. (C-E) Real-time PCR analysis of the indicated genes in cells incubated with palmitate for 6 hours in the presence/absence of S1, liPS5, cPS5 and cPS5Nal peptides. Results are presented as mean ± SEM of n=3-5 independent experiments. #p<0.05 vs basal and *p<0.05 vs PA.

### Detailed description

The present invention may be understood more readily by reference to the following detailed description of the preferred embodiments of the invention, and to the examples included therein.

Given the high prevalence and economic burden of NAFLD and the fact that no treatment is available, a better understanding of its pathogenesis is essential to identify targets for drug development to overcome the epidemic. Despite the research existing in this field and the fact that the relationship between the JAK/STAT signaling pathway and SOCS proteins has been postulated, the effective administration of a peptide per se for the prevention or treatment of an obesity related liver disorder or a pathological condition or disease caused by fatty liver has not been described up until now. SOCS mimetic peptides have not been linked in any case to such disorders.

The inventors have now found that a family of cell-permeable peptide sequences derived from the kinase inhibitory region of SOCS1 can reduce the activation of JAK/STAT pathway to relieve the development and progression of such an obesity related liver disorder, specifically NAFLD.

In summary, the effects of compound S1 peptide and its derivatives sequences PS5 (linear, cyclic and 1-naphthylalanine substitution forms) were studied in well-established models of NAFLD [10], induced by methionine and choline-deficient diet (MCD), high fat diet (HFD), leptin deficiency (BTBR ob/ob) and leptin receptor deficiency (BKS db/db). Surprisingly, JAK/STAT inhibition through theses peptides reduced hepatic transaminases and intrahepatic lipid content, and modified the expression of lipid transporters, thus improving diet induced steatosis. Treatment with the SOCS1-derived peptides of present invention also limited the content of immune cells (macrophages) and the expression of inflammatory genes (chemokines and cytokines) in the liver. Moreover, in MCD-induced NAFLD/NASH, peptide treatment prevented the expression of profibrotic genes and the accumulation of collagen fibers in hepatic tissue.

Furthermore, it could be shown that the S1 peptide suppressed the activation of STAT transcription factors in hepatic tissue and mitigates liver steatosis, hepatocellular edema and inflammatory infiltrate. The hepatoprotective effect of S1 peptide was similar or superior that the JAK1/2 inhibitor baricitinib. Lipidomic analysis revealed that S1 treatment partially reversed the alterations in the hepatic metabolic profile by reducing concentrations of fatty acids related to de novo lipogenesis. Finally, in vitro studies in hepatocytes stimulated with palmitic acid demonstrated that peptides attenuated lipid uptake and inflammation.

In conclusion, these preclinical studies showed the therapeutic potential of the SOCS1-derived peptides of present invention through suppression of liver inflammation, fibrogenesis and metabolic dysfunction, therefore attenuating the development of hepatic injury in NAFLD pathology.

The present invention therefore provides isolated polypeptides for use in the prevention, relief and/or treatment of an overweight related or obesity related liver disorder or a pathological condition or disease caused by fatty liver, specifically non-alcoholic fatty liver disease (NAFLD) or non-alcoholic steatohepatitis (NASH) or metabolic associated fatty liver disease (MAFLD).

In the present invention the term "prevention" means to avoid occurrence of the disease or pathological condition in an individual, particularly when the individual has predisposition for the pathological condition but has not yet been diagnosed.

In the present invention, the term "treat" or "treatment" comprises inhibiting the disease or pathological condition, i.e., stopping its development; relieving the disease or pathological condition, i.e., causing regression of the disease or pathological condition; relieving or reducing at least one of the symptoms or signs of the disease or pathological condition; and/or stabilizing the disease or pathological condition in an individual.

As used herein, the term "obesity related liver disorder" refers to a disease characterized by excessive fat deposition in the liver, that usually has a close relationship with overweight and can therefore also be called overweight related disorder. Exemplary obesity related disorders according to present invention are overweight/obesity or the metabolic syndrome (dyslipidemia, overweight/obesity, hypertension, insulin-resistance) or/and type 2 diabetes.

As used herein, the term "pathological condition or disease caused by fatty liver" refers to morphological (histopathological changes) and functional conditions with elevated markers of chronic liver disease (liver enzymes) related with steatosis > 5% in liver biopsy, steatohepatitis (defined by hepatocellular ballooning and inflammatory clusters), liver fibrosis (accumulation of extracellular matrix proteins including collagen) and progression to cirrhosis (end-stage liver disease with impaired liver function) or even hepatocellular carcinoma (increase of tumorigenic liver markers).

Exemplary pathological condition or disease caused by fatty liver according to present invention are steatosis, steatohepatitis, liver fibrosis or cirrhosis, more preferably non-alcoholic steatohepatitis (NASH), non-alcoholic fatty liver disease (NAFLD) and NAFLD-related cirrhosis.

In one embodiment of the present invention the isolated polypeptide comprises the sequence of SEQ ID NO.1 or SEQ ID NO.2 as shown in the below table:

| Amino acid Sequence | Sequence number |
|---|---|
| DTHFRTFRSHADYRRI | SEQ ID NO.1 |
| DTC(Acm)*RQTFRSH | SEQ ID NO.2 |

| | |
|---|---|
| *C(Acm) = acetaminomethyl-L-cysteine. | |

The non-natural amino acid acetaminomethyl-L-cysteine enhances the stability of the peptide against proteolytic degradation.

In a further embodiment the present invention relates to a variant of the polypeptide comprising the sequence of SEQ ID NO.1, which is at least 80%, 81%, 82%, 83%, 84%, 85%, 86%, 87%, 88%, 89%, 90%, 91%, 92%, 93%, 94%, 95%, 96%, 97%, 98%, 99% identical to SEQ ID NO.1.

In a further embodiment the present invention relates to a variant of the polypeptide comprising the sequence of SEQ ID NO.2, which is at least 80%, 81%, 82%, 83%, 84%, 85%, 86%, 87%, 88%, 89%, 90%, 91%, 92%, 93%, 94%, 95%, 96%, 97%, 98%, 99% identical to SEQ ID NO.2.

As used herein, the term "% of identity" or "at least % identical", in relation to amino acid sequences, means the percentage of identity determined by means of the following method: alignment of two amino acid sequences performed by means of the service provided in https://www.ebi.ac.uk/Tools/msa/clustalw2/, applying the default adjustments of this service.

In a further embodiment the isolated polypeptide further comprises at least one of the following amino acid substitutions based on the amino acid sequence of SEQ ID NO.1: H3C(Acm), F4R, R5Q, F7Nal1, S9K, A11S, a deletion of amino acids A11 to 116, or a combination thereof.

In a further preferred embodiment, the isolated polypeptide comprises SEQ ID NO.1 with at least one, at least two or at least three of the amino acid substitutions H3C(Acm), F4R and R5Q, preferred is the isolated polypeptide comprising SEQ ID NO.1 and further comprising all three amino acid substitutions H3C(Acm), F4R, R5Q as well as the deletion of A11, D12, Y13, R14, R15 and 116 (SEQ ID NO.2).

In yet a further preferred embodiment the isolated polypeptide comprises SEQ ID NO.1 with at least one, at least two, at least three, or at least four of the amino acid substitutions H3C(Acm), F4R, R5Q, S9K and the deletion of A11, D12, Y13, R14, R15 and 116 preferred is the isolated polypeptide comprising SEQ ID NO.1 and further comprising all four amino acid substitutions H3C(Acm), F4R, R5Q, S9K as well as the deletion of A11, D12, Y13, R14, R15 and 116 (SEQ ID NO.3).

In yet a further preferred embodiment the isolated polypeptide comprises SEQ ID NO.2 with at least one, at least two, at least three, or at least four of the amino acid substitutions H3C(Acm), F4R, R5Q and S9K, preferred is the isolated polypeptide comprising SEQ ID NO.1 and further comprising all four amino acid substitutions H3C(Acm), F4R, R5Q and S9K (SEQ ID NO.3).

In another preferred embodiment the isolated polypeptide comprises SEQ ID NO.1 with at least one, at least two, at least three, at least four, or at least five of the amino acid substitutions H3C(Acm), F4R, R5Q, F7Nal1 and S9K as well as the deletion of A11, D12, Y13, R14, R15 and 116, preferred is the isolated polypeptide comprising SEQ ID NO.1 and further comprising all five amino acid substitutions H3C(Acm), F4R, R5Q, F7Nal1 and S9K (SEQ ID NO.4).

In another preferred embodiment the isolated polypeptide comprises SEQ ID NO.2 with at least one, at least two, at least three, at least four, or at least five of the amino acid substitutions H3C(Acm), F4R, R5Q, F7Nal1 and S9K, preferred is the isolated polypeptide comprising SEQ ID NO.2 and further comprising all five amino acid substitutions H3C(Acm), F4R, R5Q, F7Nal1 and S9K (SEQ ID NO.4).

As herein used the amino acid "NaI1" refers to 1-naphthylalanine.

In a further embodiment of the present invention the isolated polypeptide comprises the sequence of SEQ ID NO.3 or SEQ ID NO.4 as shown in the below table:

| Amino acid Sequence | Sequence number |
|---|---|
| DTC(Acm)RQTFRKH | SEQ ID NO.3 |
| DTC(Acm)RQT(Nal1)RKH | SEQ ID NO.4 |

SEQ NO.3 and SEQ NO.4 of present invention are cyclic peptides. The sequence has a S9K substitution compared to SEQ ID NO.2 that forms a lactam bridge with the aspartic acid (at position 1) to create a cyclic peptide.

In a further embodiment the isolated polypeptide comprises a variant of SEQ IN NO.3 or SEQ ID NO.4, which is at least 80%, 81%, 82%, 83%, 84%, 85%, 86%, 87%, 88%, 89%, 90%, 91%, 92%, 93%, 94%, 95%, 96%, 97%, 98%, 99% identical to SEQ IN NO.3 or SEQ ID NO.4, respectively.

In a preferred embodiment the polypeptide consists of
i. the amino acid sequence of SEQ ID NO.1; or
ii. the amino acid sequence of SEQ ID NO.2; or
iii. the amino acid sequence of SEQ ID NO.3; or
iv. the amino acid sequence of SEQ ID NO.4.

In a further preferred embodiment, the polypeptide consists of
a) the murine SOCS1 protein (UniProt: 035716);
b) the human SOCS1 protein (UniProt: 015524); or
c) a variant of the sequence of a) or b) which is at least 80% homologous to the amino acid sequence of the murine SOCS1 protein or to the amino acid sequence of the human SOCS1 protein.

One or more of the amino acids of any of the polypeptide sequences of present invention, particularly SEQ ID NO. 1, SEQ ID NO. 2, SEQ ID NO. 3 and SEQ ID NO. 4 can be modified, for example, they can be phosphorylated. According to a particular embodiment, only one amino acid of the sequence is modified, preferably phosphorylated. According to a preferred embodiment, the phosphorylated amino acid is the tyrosine (Y).

Likewise, the polypeptides of present invention can be bound to a cell permeability region, preferably to a palmitate-lysine group. According to a preferred embodiment, the cell permeability region is bound at the N-terminal end of the polypeptide, the cell permeability region being more preferably a palmitate-lysine group. In one preferred embodiment the polypeptide consists of SEQ ID NO.1, wherein the polypeptide is bound to a cell permeability region.

In another preferred embodiment of present invention, the polypeptides of present invention, preferably the polypeptides as defined in SEQ ID NO.2, 3 and 4 are conjugated to a sequence derived from the transactivator of transcription (TAT) of human immunodeficiency virus, GRKKRRQRRRPPQGG. This sequence assists in cell penetration.

As shown in Example 1, the effects of the SOCS-1 derived peptides S1 peptide (SEQ ID NO.1) and its derivative containing a non-natural residue Cys(Acm) (liPS5 peptide; SEQ ID NO.2) were studied by the inventors and it was found that circulating levels of transaminase GPT, a liver damage indicator, were substantially elevated in mice fed with methionine-choline deficient diet (MCD) when compared with mice with normal chow diet and were significantly reduced by S1 and liPS5 administration (Fig. 1C). In addition, peptide treatment partially reversed the decrease of serum cholesterol (Fig. 1D) and triglycerides (Fig. 1E) levels associated to MCD diet.

The inhibitory effect of the S1 peptide (SEQ ID NO.1) and the liPS5 peptide (SEQ ID NO.2) on the activation of JAK/STAT pathway in mouse liver was assessed and the inventors have found that their administration decreased P-STAT1 and P-STAT3 induced by MCD diet at 3 and 5 weeks (Fig. 2A-C).

The pathological evaluation of liver sections revealed that MCD diet induces steatosis (macro and microvesicular), hepatocyte ballooning and lobular inflammation which could be significantly reduced in S1- and liPS5-treated groups (Fig. 3A and B). Furthermore, in mice with induced hepatic steatosis significant differences in the hepatic lipid content between control vehicle and S1/liPS5 groups were found (Fig. 4A and B).

Peptide treatment also diminished the concentration of total cholesterol (Fig. 5A) and TG (Fig. 5B) in liver tissues. Moreover, S1/liPS5 administration downregulated the expression of CD36, a scavenger receptor involved in fatty acid uptake (Fig. 5C), and upregulated ATP bind cassette transporter G1 (ABCG1), a reverse cholesterol transporter (Fig. 5D). These findings indicate a partial reversion of hepatic lipid accumulation by peptides.

To assess the impact of SOCS1-derived peptides on liver inflammation, the inventors determined macrophage accumulation and cytokine expression in liver samples (Fig. 6). Compared with control chow group, mice fed with MCD diet showed significantly increased F4/80 positive staining that was reduced by administration of S1 and liPS5 peptides (Fig. 6, A and B). Both treatments significantly reduced the gene expression of the chemokines C-C motif chemokine ligand 2 (CCL2; Fig. 6C), CCL5 (Fig. 6D) and C-X-C motif chemokine 10 (CXCL10; Fig. 6E), and the pro-inflammatory cytokines interleukin (IL-) 6 (Fig. 6F), IL-1β (Fig. 6G), and tumor necrosis factor α (TNFα; Fig. 6H) induced by MCD diet.

Hepatic collagen was evaluated by picrosirius red staining (Fig. 7A). Results indicate an increase in the liver collagen content in mice fed with MCD diet as compared with control chow mice, and a partial and significant reversion by S1 and liPS5 treatment (Fig. 7B). In line with this, the gene expression of pro-fibrotic markers collagen 1α (COL1α; Fig. 7C), actin α2 (ACTA2; Fig. 7D) and transforming growth factor β (TGFβ; Fig. 7E) were also found reduced by S1 and liPS5.

The inventors could therefore show that the SOCS1-derived peptides or present invention reduce hepatic steatosis, inflammation, and fibrosis in a NAFLD/NASH mouse model induced by methionine-choline deficient diet.

In one preferred embodiment of present invention the pathological condition or disease is therefore an obesity related liver disorder selected from steatosis, steatohepatitis, liver fibrosis or cirrhosis, more preferably non-alcoholic steatohepatitis (NASH), non-alcoholic fatty liver disease (NAFLD) and NAFLD related cirrhosis.

In a preferred embodiment of present invention the obesity related liver disorder is non-alcoholic fatty liver disease (NAFLD).

In a further aspect present invention relates to a composition, preferably a pharmaceutical composition, comprising a therapeutically effective amount of a polypeptide as defined herein and at least one pharmaceutically acceptable vehicle or excipient, for use in the prevention, relief and/or treatment of an obesity related liver disorder or a pathological condition or disease caused by fatty liver which is preferably selected from steatosis, steatohepatitis, liver fibrosis or cirrhosis, non-alcoholic steatohepatitis (NASH), non-alcoholic fatty liver disease (NAFLD) and NAFLD related cirrhosis.

In the compositions for use according to the invention, the polypeptide can be contained in a concentration range of 1-12 mg/mL. In the particular case of parenteral administration, the polypeptide will be contained in a concentration between 1 and 5 mg/mL, in particular embodiments it will be contained in a concentration between 1 and 3 mg/mL, according to a preferred embodiment, in a concentration of 2 mg/mL ± 10% or ± 5%, i.e., ± 0.2 mg/mL or ± 0.1 mg/mL.

The composition for use according to the invention is suitable for the administration of a daily dose of between 1 and 16 mg of polypeptide. The composition for use according to the invention is also suitable for the administration of a dose of between 1 and 16 mg of polypeptide every two or every three days. In the case of parenteral administration, the composition will be suitable for the administration of a daily dose of between 2 and 16 mg/kg of weight of the patient or subject on which administration is performed, according to particular embodiments, between 4 and 10 mg/kg of weight of the patient or subject. The parenteral administration of between 2 and 16 mg/kg or 4 and 10 mg/kg of weight of the patient or subject can also be performed every two or every three days.

In one embodiment the compositions for use according to the present invention comprise at least one pharmaceutically acceptable vehicle or excipient.

The term "pharmaceutically acceptable vehicle or excipient" as used herein refers to molecular substances or entities together with which the peptide of the invention is administered and will be chosen according to the route of administration. The vehicles can be sterile liquids, such as water or oils, including those derived from petroleum, those of animal, plant or synthetic origin, excipients, disintegrating agents, wetting agents or diluents. Suitable vehicles and excipients are described, for example in "Remington's Pharmaceutical Sciences" of E.W. Martin.

The compositions for use according to the present invention can be administered through any known route, including orally, gastroenterically, parenterally, rectally and by respiratory, preferred is the parenteral route of administration. Likewise, the compositions may contain other suitable active ingredients or adjuvants.

In a further preferred embodiment, the compositions may contain one type of polypeptide according to the invention or a mixture of two or more polypeptides according to the invention.

In one embodiment the composition comprises a polypeptide of SEQ ID NO.1 and a polypeptide of SEQ ID NO.2, or any of their variants as described herein above.

In another embodiment the composition comprises a polypeptide of SEQ ID NO.1 and a polypeptide of SEQ ID NO.3, or any of their variants as described herein above.

In another embodiment the composition comprises a polypeptide of SEQ ID NO.1 and a polypeptide of SEQ ID NO.4, or any of their variants as described herein above.

In another embodiment the composition comprises a polypeptide of SEQ ID NO.2 and a polypeptide of SEQ ID NO.3, or any of their variants as described herein above.

In another embodiment the composition comprises a polypeptide of SEQ ID NO.2 and a polypeptide of SEQ ID NO.4, or any of their variants as described herein above.

In another embodiment the composition comprises a polypeptide of SEQ ID NO.3 and a polypeptide of SEQ ID NO.4, or any of their variants as described herein above.

The peptides of the present invention can be used alone or in combination, or in combination with other agents having a metabolic activity. For example, these agents may be selected from antioxidants (e.g. vitamin E), glucagon-like peptide 1 (GLP-1) analogues (e.g. semaglutide), dual glucose-dependent insulinotropic peptide (GIP) and GLP-1 receptor agonist (e.g tirzepatide), sodium-glucose cotransporter 2 inhibitors (e.g. empaglifozin, dapaglifozin, canagliflozin and other members of the family), as well other novel anti-hyperglycemic and anti-obesity drugs.

In one embodiment the pharmaceutical composition therefore further comprises at least one other active ingredient.

Throughout the description and claims the word "comprise" and its variants are not intended to exclude other technical characteristics, additives, components or steps. For those skilled in the art, other objects, advantages and characteristics of the invention will become apparent in part from the description and partly from the practice of the invention. The following examples and figures are provided by way of illustration and are not intended to limit the scope of the present invention.

The sequences SEQ ID NOs.1 to 4 are also depicted in the sequence listing attached to the present description. In case of discrepancy between the content of the attached sequence listing and the sequences as depicted in the tables of present description, the sequences as depicted in the tables of present description shall be deemed the correct sequences.

### EXAMPLES

### MATERIAL AND METHODS

### 1. Peptide synthesis

Four active peptide sequences derived from SOCS1 KIR domain (residues 53-68) were studied: S1, linear PS5 (liPS5), cyclic PS5 (cPS5), and cyclic with 1-naphthylalanine (cPS5Nal).

The sequences of the four peptides as well as the amino acid exchanges made based on S1 as the reference peptide are shown in the table below (numbering from N to C terminal end):

| Peptide name | Amino acid Sequence | Sequence number | Amino acid exchanges with reference to S1 |
|---|---|---|---|
| S1 | DTHFRTFRSHADYRRI | SEQ ID NO.1 | - |
| linear PS5 (liPS5) | DTC(Acm)RQTFRSH | SEQ ID NO.2 | H3C(Acm), F4R, R5Q |
| cyclic PS5 (cPS5) | DTC(Acm)RQTFRKH (lactam bridge D1-K9) | SEQ ID NO.3 | H3C(Acm), F4R, R5Q, S9K |
| cyclic PS5 with 1-naphthyl-alanine (cPS5Nal) | DTC(Acm)RQT(Nal1)RKH (lactam bridge D1-K9) | SEQ ID NO.4 | H3C(Acm), F4R, R5Q, F7Nal1, S9K |

Furthermore, amino acids ADYRRI (positions 11 to 16), i.e. A11 to I16, have been deleted in the peptides as depicted by SEQ ID NO.2 to 4 with reference to the S1 peptide as depicted by SEQ ID NO.1.

An inactive mutant form of S1 (Mut, Phe->Ala substitutions) was included as negative control. For in vivo and in vitro studies, SOCS1 sequences were conjugated at N-terminal with the cell penetrating sequences palmitoyl-Lys or transactivator of transcription of human immunodeficiency virus (TAT). Peptides were synthesized by solid phase method following the Fmoc strategy, purified by preparative reverse phase-HPLC and lyophilized. Peptides were dissolved in 1% DMSO in saline solution (S1 and Mut) and H₂O (liPS5, cPS5 and cPS5Nal), then filter-sterilized and stored at -80°C before use.

### 2. Experimental models of NAFLD

All the procedures carried out in this study were performed mice (Mus musculus) under the principle for replacement, refinement or reduction (the 3Rs) and in accordance with the Directive 2010/63/EU of the European Parliament and were approved by the Institutional Animal Care and Use Committee of IIS-Fundacion Jimenez Diaz and Comunidad de Madrid (PROEX 079/18 and 217/19).

### 2.1. Dietary models of NAFLD/NASH

In the first model, C57BL/6J mice (males, 20 weeks old) were administered methionine-choline deficient diet (MCD, TD.90262, Envigo) for 1 week, and then randomly distributed to receive vehicle or peptides (S1 and liPS5) at the dose of 4 µg/g (in 200 □L) via intraperitoneal injection 3 days per week for additional 2 and 4 weeks (Veh, n=8; S1,n=5; liPS5, n=7; each time). A group of mice under standard diet (Chow, n=6) was included as control.

In the second model, apolipoprotein E deficient mice (ApoE-/-, males, 10-12 weeks old) were administered high-fat diet (HFD, TD.88137, Envigo) for 8 weeks and treated with vehicle (n=8) or SOCS1-derived peptides (S1, n=6; liPS5, n=6; cPS5, n=6; cPS5Nal, n=6) at the dose of 4µg/g (in 200 µL) via intraperitoneal injection 3 days per week. A group of ApoE-/- mice under standard diet (Chow, n=6) was included as control.

### 2.2. Genetic NAFLD models associated to obesity and T2D

Leptin deficiency in diabetes-susceptible strain BTBR ob/ob: Black and tan, brachyuric (BTBR) obese/obese (ob/ob) mice (males, 6 weeks-old) were randomized to receive vehicle (n=7), S1 peptide (2 and 4 µg/g body weight; n=7) or mutant inactive peptide (Mut, 4 µg/g body weight; n=7) via intraperitoneal injection 3 days per week for 7 weeks. In a second experiment, BTBR ob/ob mice (males, 16 weeks old) were treated with vehicle (n=8), S1 peptide (10 µg/g body weight; n=6) or JAK1/2 inhibitor (baricitinib, 3 µg/g body weight; n=6) for 7 weeks. A group of BTBR wild-type mice (WT, n=6) was included as control.

Leptin receptor deficiency in diabetes-susceptible strain BKS db/db: Homozygous C57BLKS (BKS) inbred db/db mice (16-weeks old males; Envigo) were randomized to receive in drinking water an angiotensin receptor blocker (Losartan 25 µg/g body weight) or vehicle as tap water (n=25). In addition, two groups of 8 losartan-treated mice were randomized to receive S1 peptide (10 µg/g body weight) or JAK1/2 inhibitor (Baricitinib 3 µg/g body weight) via intraperitoneal injection 3 days per week for 7 weeks. All groups were monitored during 7 weeks of treatment. Heterozygous littermates (db/m; n=10) were used as non-diabetic, non-obese control group.

Mice were housed in ventilated cages (2-4 mice per cage) with usual bedding material and environmental enrichment in a conventional temperature-controlled room (20-22°C) with 12 h light/dark cycle and free access to water and food. Blood glucose (glucometer; NovaPro, Menarini Diagnostics) and body weight levels were monitored once weekly during the follow-up period. At the end of the intervention studies, mice were anaesthetized (ketamine 100 mg/kg and xylazine 10 mg/kg) and euthanized, and blood and tissue samples were collected.

### 3. Biochemical analysis in serum and liver

At the end of the studies, serum levels of glucose, triglycerides (TG), total cholesterol, high-density lipoprotein cholesterol (HDL), low-density lipoprotein cholesterol (LDL), alanine aminotransferase (ALT or GPT), aspartate aminotransferase (AST), alkaline phosphatase (ALP) and albumin were determined by automated procedures (Roche Cobas autoanalyzer). In some serum samples, GPT activity was determined using Reflotron strips (Roche Diagnostics, Barcelona).

Hepatic lipids were extracted as previously described [24]. Commercial colorimetric kits were used to measure concentrations of total cholesterol (STA-384, Cell Biolabs) and TG (11528, Biosystems), and values were normalized by tissue weight and mg of protein (BCA assay), respectively.

A lipidomic assay was performed in liver samples from BTBR ob/ob mice (22 weeks old). Briefly, TG and non-esterified fatty acids (NEFA) were isolated by solid-phase extraction [25]. Fatty acids were hydrolyzed and methylated [26] and then analyzed by gas chromatography/electron ionization mass spectrometry (GC/MSEI), using an Agilent 6890N GC equipped with an Agilent 7683 autosampler, and an Agilent 5973N mass spectrometry detector. Fatty acid methyl esters (FAME) were separated with a J&W DB-FastFAME capillary column (30 m x 0.2 mm x 0.25 µm film thickness) (Agilent). The concentrations of FAME in the samples were calculated by linear regression of the peak area ratio relative to that of the internal standard, and values were normalized by tissue weight.

### 4. Histological and immunohistochemical analysis

At the time of harvest, liver samples were fixed in 10% buffered formalin, processed for paraffin-embedding and cut (3-4 µm) or embedded in OCT compound and cryosectioned into 7 µm slices. Deparaffinized sections were stained with hematoxylin/eosin and Masson's trichrome to evaluate the histological NAFLD activity score (NAS) considering steatosis, lobular inflammation, ballooning and fibrosis, as previously described [27] or were stained with picrosirious red to assess collagen deposition and fibrotic areas. Periodic Acid Schiff staining was performed for glycogen deposition assessment. Cryosections were stained with Oil red O/ hematoxylin to detect lipid accumulation.

For immunohistochemistry, liver sections were deparaffinized and dehydrated through graded xylene and ethanol. After antigen retrieval (0.01 M citrate buffer pH 6 for 20 min) and blockade of endogenous peroxidase (3% H2O2 in methanol for 30 min) and nonspecific binding (8% host serum for 30 min), slides were incubated overnight at 4°C with primary antibodies against phosphorylated STAT1 (P-STAT1, Tyr701; Thermo Fisher Scientific Cat# 44-376G, RRID:AB_2533642), P-STAT3 (Ser727, Cell Signalling Technology Cat# 9134, RRID:AB_331589), P-nuclear factor erythroid 2-related factor 2 (NRF2, Ser40; Abcam Cat# ab76026, RRID:AB_1524049) and F4/80 monocytes/macrophages (Bio-Rad Cat# MCA497R, RRID:AB_323279). After rinsing in PBS, samples were incubated with respective secondary antibodies conjugated with either horseradish peroxidase or biotin (followed by avidin-biotin complex reagent). Immunoreactive cells were then visualized by the addition of peroxidase substrates (3, 3-diaminobenzidine or 3-amino-9-ethylcarbazole; DAKO) and counterstained with hematoxylin.

All the histological evaluations were conducted in a blinded fashion. Positive staining was quantified in at least 2 sections per mice using Image-Pro Plus software (Media Cybernetics) and expressed as percentage or number of positive cells per area.

### 5. Cell cultures

Immortalized mouse hepatocyte cell line (kindly provided by Dr. Valverde, IIBm Alberto Sols, Madrid) were cultured in Dulbecco's Modified Eagle Medium (DMEM; D6546, Sigma-Aldrich) containing 10% FBS, 100 U/mL penicillin, 100 µg/mL streptomycin and 2 mM L-glutamine (Sigma-Aldrich) and used between 2nd-8th passages [28]. Cells were incubated for 24 hours in medium with 0.5% FBS, pre-treated for 90 min with optimal doses of peptides (59 µM S1 and 25 µM liPS5) and then stimulated for additional 2-16 h with palmitic acid (P-0500, Sigma-Aldrich; stock solution diluted in fatty acid-free BSA to 5 mM) at a final concentration of 400 µM. Cell viability was confirmed by the 3-(4,5-dimethylthiazol-2-yl)-2,5-diphenyltetrazolium bromide tetrazolium (MTT) assay. Lipid accumulation in hepatocytes was determined by Oil red O staining.

### 6. Real time PCR analysis

Total RNA from mouse liver and hepatocytes was extracted using TRI Reagent (Molecular Research Center). Complementary DNA was produced via reverse transcription using High Capacity cDNA Reverse Transcription Kit (Applied Biosystems). The gene expression of mouse genes was determined in duplicate by quantitative real-time PCR using predesigned TaqMan gene expression assays (Applied Biosystems) or Premix ExTaq (Takara). The results for each sample were normalized to the 18S rRNA housekeeping expression, and data expressed as fold increases versus chow diet (in vivo studies) or basal conditions (in vitro studies).

### 7. Protein expression analysis

Approximately 30 µg of protein from mouse tissue and cell lysates were electrophoresed, then transferred to PVDF membranes and immunoblotted for P-STAT1 (Tyr701; Innovative Research Cat# 33-3400, RRID:AB_87095), P-STAT3 (Ser727; Cell Signalling Technology Cat# 9134, RRID:AB_331589), STAT1 (Cell Signalling Technology Cat# 9172, RRID:AB_2198300), STAT3 (Cell Signalling Technology Cat# 9139, RRID:AB_331757), β-actin (Sigma-Aldrich Cat# A1978, RRID:AB_476692) and β-tubulin (Sigma-Aldrich Cat# T5168, RRID:AB_477579). Densitometric data were normalized to loading control (β-actin or β-tubulin) and expressed as fold increases versus chow mice (in vivo studies) or basal conditions (in vitro studies).

### 8. Statistical analysis

Results are presented as individual data and/or mean ±SEM from separate animals and cell experiments. In all the analysis, technical replicates were averaged to provide a single value per each biological replicate. Statistical analyses were performed using GraphPad v.5 (GraphPad Software Inc., La Joya, CA). Data passed the D'Agostino and Pearson omnibus normality test and were tested for homogeneity of variance with the Bartlett test. Pearson's correlation analysis was performed for normally distributed parameters. Statistical significance was set at p<0.05 using unpaired two-tailed Student's t test, one-way ANOVA with Bonferroni pairwise comparison test or two-way ANOVA with Tukey's multiple comparisons test. Results expressed as fold changes versus chow or basal conditions to avoid unwanted sources of variation, were subjected to non-parametric Mann-Whitney test or Kruskal-Wallis test with by Dunn's multiple comparisons test.

### EXAMPLE 1: SOCS1-derived peptides reduce hepatic steatosis, inflammation, and fibrosis in the NAFLD/NASH mouse model induced by methionine-choline deficient diet

The effects of S1 peptide and its derivative containing a non-natural residue Cys(Acm) (liPS5 peptide) were studied in WT mice fed on MCD for 3 and 5 weeks. During the study period, no differences in body weight (Fig. 1A) and glycemia (Fig. 1B) levels were observed in peptide treated mice when compared with vehicle control group. At the end of the study, circulating levels of transaminase GPT (liver damage indicator; Fig. 1C) were substantially elevated in mice fed with MCD when compared with mice with normal chow diet; and were significantly reduced by S1 and liPS5 administration (% vs Veh: 60% at 3weeks, 75% at 5 weeks). In addition, peptide treatment partially reversed the decrease of serum cholesterol (Fig. 1D) and triglycerides (Fig. 1E) levels associated to MCD diet.

The inhibitory effect of SOCS-1 derived peptides on the activation of JAK/STAT pathway in mouse liver was assessed by Western blot (Fig. 2A) and immunohistochemistry (Fig. 2, B-C) of phosphorylated STAT1/3 proteins (P-STAT1 and P-STAT3) in liver samples. Results indicate that the administration of S1 and liPS5 decreased P-STAT1/ and P-STAT3 levels induced by MCD diet at 3 and 5 weeks.

Pathological evaluation of liver sections stained with hematoxilin/eosin revealed that MCD diet induces steatosis (macro and microvesicular), hepatocyte ballooning and lobular inflammation (Fig. 3A). The results of NAS score for each group revealed significant reduction in S1- and liPS5-treated groups as compared with vehicle control group, both at 3 and 5 weeks of MCD diet (Fig. 3B).

Hepatic steatosis was further assessed by Oil red O staining, a histological marker of fat accumulation (Fig. 4A). Compared to mice with normal chow diet, mice with MCD diet had significantly increases in the Oil red O positive area; however, significant differences in the hepatic lipid content between control vehicle and S1/liPS5 groups were found (Fig. 4B). Peptide treatment also diminished the concentration of total cholesterol (Fig. 5A) and TG (Fig. 5B) in liver tissues. Moreover, quantitative real-time PCR analysis showed that that S1/liPS5 administration downregulated the expression of CD36, a scavenger receptor involved in fatty acid uptake (Fig. 5C), and upregulated ATP bind cassette transporter G1 (ABCG1), a reverse cholesterol transporter (Fig. 5D). These findings indicate a partial reversion of hepatic lipid accumulation by peptides.

To assess the impact of SOCS1-derived peptides on liver inflammation, we determined macrophage accumulation and cytokine expression in liver samples (Fig. 6). Compared with control chow group, mice fed with MCD diet showed significantly increased F4/80 positive staining that was reduced by administration of S1 (% vs Vehicle: 45% at 3weeks; 38% at 5 weeks) and liPS5 (% vs Vehicle: 45% and 62%) peptides (Fig. 6, A and B). Moreover, transcriptional analysis revealed that both treatments significantly reduced the gene expression of the chemokines C-C motif chemokine ligand 2 (CCL2; Fig. 6C), CCL5 (Fig. 6D) and C-X-C motif chemokine 10 (CXCL10; Fig. 6E), and the pro-inflammatory cytokines interleukin (IL-) 6 (Fig. 6F), IL-1β (Fig. 6G), and tumor necrosis factor α (TNFα; Fig. 6H) induced by MCD diet.

Hepatic collagen was evaluated by picrosirius red staining (Fig. 7A). Results indicate an increase in the collagen content in mice fed with MCD diet as compared with control chow mice, and a partial and significant reversion by S1 and liPS5 treatment (Fig. 7B). In line with this, the gene expression of pro-fibrotic markers collagen 1α (COL1α; Fig. 7C), actin α2 (ACTA2; Fig. 7D) and transforming growth factor β (TGFβ; Fig. 7E) were also found reduced in S1 and liPS5 groups when compared with vehicle group.

### EXAMPLE 2: SOCS1-derived peptides prevent the development of NAFLD induced by high-fat diet in mice

Given that ApoE is an important player in lipoprotein metabolism and its absence predisposes to hypercholesterolemia, atherosclerosis, obesity and NAFLD [29], we characterized the actions of SOCS1-derived peptides in ApoE-/- mice fed with HFD. This model mimics metabolic syndrome features more closely than MCD model, and presents major characteristics of human NASH including steatosis, inflammation and fibrosis. To improve the kinetic affinity of liPS5 and its resistance to protease degradation, two cyclic analogues containing a lactam bridge between side chains of aspartic acid and lysine (cPS5) and an additional non-natural residue 1-naphthylalanine (cPS5Nal) were designed and structurally characterized [30]. Herein, we characterized in vivo the biological effect of the therapy of S1 and its derivatives (liPS5, cPS5 and cPS5Nal) in ApoE-/- mice fed with a HFD containing 42% fat, 0.2% cholesterol. After 8 weeks of treatment, pathological evaluation of liver sections stained with hematoxylin/eosin, Oil red O and F4/80 revealed a marked decrease of hepatic steatosis, ballooning and inflammation in mice treated with S1, liPS5, cPS5 and cPS5Nal peptides when compared with mice receiving vehicle (Fig. 8, A, C and D). Hepatic content of triglycerides was also significantly reduced in the four groups of treated mice (Fig. 8B). In addition, quantitative real-time PCR analysis showed that S1, liPS5, cPS5 and cPS5Nal peptides effectively prevented the gene expression of the scavenger receptor CD36 (Fig. 9A), the chemokines CCl2, CCL5 and CXCL10 (Fig. 9, B-D) and the pro-inflammatory cytokines IL-6 and TNFα (Fig. 9, E and F) in mouse liver.

### EXAMPLE 3: SOCS1-derived peptide ameliorates NAFLD in a mouse model of obesity and type 2 diabetes

Given that NAFLD development in patients is often associated with obesity and T2D, we aimed to determine the actions of SOCS1-derived peptide in BTBR ob/ob mice, a model characterized by progressive development of obesity and hyperglycemia leading to renal dysfunction and hepatic damage [22, 31]. To explore the impact of S1 peptide on early NAFLD development, BTBR ob/ob at 6 weeks of age were administered S1 peptide for 7 weeks, using vehicle and inactive mutant peptide (Mut) as control groups. There were no differences in body weight (Fig. 10A) and blood glucose (Fig. 10B) among the mouse groups along the study. At the end of the experimental period, histopathological analysis revealed the presence of micro/macrosteatosis, inflammatory cells and hepatocellular ballooning in liver sections of BTBR ob/ob mice (Fig. 10C). Compared with vehicle group, mice receiving two different doses of S1 peptide exhibited a partial reversion of NAFLD histological features, whereas the inactive Mut peptide was ineffective (Fig. 10D). Moreover, P-STAT3 immunohistochemistry confirmed a dose-dependent inhibitory effect of S1 peptide on JAK/STAT pathway activation in damaged liver (Fig. 11, A and B). Similarly, the reduced levels of P-NRF2 protein found in S1-treated groups (Fig. 11, A and C) suggest inhibition of Keap-NRF2 pathway, an endogenous redox signaling pathway with a double-edged sword role in NAFLD progression whose activation is linked to insulin resistance and hepatic steatosis [32].

We further explored whether targeting JAK/STAT pathway can slow down the progression of NAFLD. In a second set of experiments, BTBR ob/ob mice at 16 weeks of age were treated with S1 peptide or JAK1/2 inhibitor baricitinib for 7 weeks. At study termination, we did not observe any significant changes in biochemical and metabolic parameters in S1-treated mice when compared with vehicle group (Fig. 12, A-C), except for LDL and urea (Fig. 12C). Histological evaluation confirmed a marked decrease in total NAS score by S1 peptide, which produced a superior effect than baricitinib on lobular inflammation and ballooning (Fig. 13, A-D). In this model we performed a lipidomic analysis of intrahepatic TG and NEFA fractions focusing on the major products of de novo lipogenesis. Hierarchical clustering and partial least squares discriminant analysis showed distinctive lipid profiles between vehicle and treated mice (Fig. 13). In the hepatic TG fraction, significant lower levels of C16:0 (palmitic), C16:1n-7 (palmitoleic), and C18:1n-9cis (oleic) were observed in S1 group, but not in baricitinb group (Fig. 14, A and B). Both groups of treatment also exhibited a tendency to increase polyunsaturated fatty acids (Fig. 14, C-E), with significant differences in C20:3n6 (dihomo γ-linoleic) and C20:4n6 (arachidonic) occurring in S1-treated mice.

Finally, in a second model of T2D-induced liver damage, the BKS db/db mice with gene deficiency in leptin receptor, we studied the effects of administration of the antihypertensive drug losartan in combination with peptide S1 or baricitinib for 7 weeks. Losartan alone did not produce an hepatoprotective effect in mice, but interestingly, the combined treatment of losartan with S1 peptide or baricitinib significantly reduced hyperglycemia (Fig. 16A) without affecting body weight (Fig. 16B), and also improved NAFLD histological features (e.g. steatosis, ballooning and total NAS score; Fig. 16C and 17) and biochemical parameters (Fig. 18) when compared with untreated BKS db/db mice.

### EXAMPLE 4: In vitro effects of SOCS1-derived peptides in hepatocytes

To corroborate the in vivo findings, an in vitro model of NAFLD was developed by exposing mouse hepatocyte cell line to physiological doses of palmitate, a common long chain fatty acid in the Western diet and most abundant in liver of NAFLD patients and animal models [33]. In these studies, cells were treated with a solution of palmitic acid complexed to BSA at a final concentration of 400 µM, both in the presence and absence of peptides. These experimental conditions did not affect cell viability, as confirmed by MTT assay (not shown). Western blot analysis showed that S1 and liPS5 peptides prevented the phosphorylation of STAT1 protein in hepatocytes exposed to palmitic acid (Fig. 16A). Moreover, Oil red O staining of cells exposed to fatty acid for 2 hours revealed significant accumulation of neutral lipid droplets, and this effect was significantly decreased in the presence of peptides (Fig. 16B). Finally, by real-time PCR analysis we observed that S1 leader compound and its derivatives (liPS5, cPS5 and cPS5Nal) downregulated the expression of scavenger receptor CD36 and inflammatory genes CCL2 and IL-6 induced by palmitic acid in hepatocytes.

### List of References:

1. European Association for the Study of the Liver et al. EASL-EASD-EASO clinical practice guidelines for the management of non-alcoholic fatty liver disease. J Hepatol. 2016;64:1388-1402.
2. Younossi ZM et al. The economic and clinical burden of NAFLD in the US and Europe. Hepatology. 2016;64:1577-86.
3. Estes C, et al. Modeling NAFLD disease burden in China, France, Germany, Italy, Japan, Spain, United Kingdom, and United States for the period 2016-2030. J Hepatol. 2018;69(4):896-904.
4. Marengo A et al. Progression and natural history of NAFLD in adults. Clin Liver Dis. 2016;20:313-324.
5. Fotbolcu H et al. Nonalcoholic fatty liver disease as a multi-systemic disease. World J Gastroenterol. 2016;22:4079-90.
6. Satiya J, et al. Narrative review of current and emerging pharmacological therapies for nonalcoholic steatohepatitis. Transl Gastroenterol Hepatol. 2021;6:60.
7. Diehl AM, et al. Cause, Pathogenesis, and Treatment of Nonalcoholic Steatohepatitis. N Engl J Med 2017; 377:2063-2072.
8. Huby T, et al. Immune cell-mediated features of non-alcoholic steatohepatitis. Nat Rev Immunol. 2021 Nov 5;1-15.
9. Finck BN. Targeting Metabolism, Insulin Resistance, and Diabetes to Treat Nonalcoholic Steatohepatitis. Diabetes. 2018;67:2485-2493.
10. Stephenson et al. Updates on Dietary Models of Nonalcoholic Fatty Liver Disease: Current Studies and Insights. Gene Expr. 2018;18: 5-17.
11. Kaltenecker D, et al. Hepatic growth hormone-JAK2-STAT5 signalling: Metabolic function, non-alcoholic fatty liver disease and hepatocellular carcinoma progression. Cytokine. 2019 Dec; 124: 154569.
12. Shi SY et al. JAK2 Dissociates Hepatosteatosis from Hepatocellular Carcinoma in Mice. J Biol Chem. 2017;292:3789-3799.
13. Dodington DW et al. JAK/STAT- Emerging players in metabolism. Trends Endocrinol Metab. 2018;29:55-65.
14. Grohmann M, et al. Obesity Drives STAT-1-Dependent NASH and STAT-3-Dependent HCC. Cell. 2018;175(5):1289-1306.e20.
15. Zhou C, et al. Overexpression of miR-142-5p inhibits the progression of nonalcoholic steatohepatitis by targeting TSLP and inhibiting JAK-STAT signaling pathway. Aging (Albany NY). 2020;12(10):9066-9084.
16. Shi SY, et al. Janus Kinase 2 (JAK2) Dissociates Hepatosteatosis from Hepatocellular Carcinoma in Mice. J Biol Chem. 2017;292(9):3789-3799.
17. Recio C et al. Gene delivery of suppressors of cytokine signaling (SOCS) inhibits inflammation and atherosclerosis development in mice. Basic Res Cardiol. 2015;110:8.
18. Lopez-Sanz L et al. SOCS1-targeted therapy ameliorates renal and vascular oxidative stress in diabetes via STAT1 and PI3K inhibition. Lab Invest. 2018;98:1276-1290.
19. Bernal S, et al. Protective effect of suppressor of cytokine signalling 1-based therapy in experimental abdominal aortic aneurysm. Br J Pharmacol. 2021 Feb;178(3):564-581.
20. Recio C et al. Suppressor of Cytokine Signaling-1 Peptidomimetic Limits Progression of Diabetic Nephropathy. J Am Soc Nephrol. 2017;28:575-585.
21. La Manna S et al. Antioxidant Effects of PS5, a Peptidomimetic of Suppressor of Cytokine Signaling 1, in Experimental Atherosclerosis. Antioxidants (Basel). 2020 Aug 14;9(8):754.
22. Opazo-Ríos L, et al. Anti-inflammatory, antioxidant and renoprotective effects of SOCS1 mimetic peptide in the BTBR ob/ob mouse model of type 2 diabetes. BMJ Open Diabetes Res Care. 2020 Sep;8(1):e001242.
23. Clee SM et al. Genetic and genomic studies of the BTBR ob/ob mouse model of type 2 diabetes. Am J Ther. 2005;12:491-498.
24. Bligh E.G., Dyer W.J. A rapid method of total lipid extraction and purification. Canadian Journal of Biochemistry and Physiology. 1959;37:911-917.
25. Burdge GC, et al. A method for separation of phosphatidylcholine, triacylglycerol, non-esterified fatty acids and cholesterol esters from plasma by solid-phase extraction. Br J Nutr. 2000;84:781-787.
26. Agren J, et al. Rapid separation of serum lipids for fatty acid analysis by a single aminopropyl column. J Lipid Res. 1992;33:1871-1876.
27. Liang W, et al. Establishment of a general NAFLD scoring system for rodent models and comparison to human liver pathology. PloS One 2014;9(12):e115922.
28. Nevado C, et al. Role of insulin receptor and balance in insulin receptor isoforms A and B in regulation of apoptosis in simian virus 40-immortalized neonatal hepatocytes. Mol Biol Cell. 2008 Mar;19(3):1185-98.
29. Ferré, N. et al. Increased susceptibility to exacerbated liver injury in hypercholesterolemic ApoE-deficient mice: potential involvement of oxysterols. Am J Physiol Gastrointest Liver Physiol. 2009;296: G553-562.
30. La Manna S, et al. Cyclic mimetics of kinase-inhibitory region of Suppressors of Cytokine Signaling 1: Progress toward novel anti-inflammatory therapeutics. Eur J Med Chem. 2021;221:113547.
31. Lan H, et al. Gene expression profiles of nondiabetic and diabetic obese mice suggest a role of hepatic lipogenic capacity in diabetes susceptibility. Diabetes. 2003;52(3):688-700.
32. Wang YL, et al. A double-edged sword: The Kelch-like ECH-associated protein 1-nuclear factor erythroid-derived 2-related factor 2-antioxidant response element pathway targeted pharmacological modulation in nonalcoholic fatty liver disease. Curr Opin Pharmacol. 2021;60:281-290.
33. Hetherington AM, et al. Differential Lipotoxic Effects of Palmitate and Oleate in Activated Human Hepatic Stellate Cells and Epithelial Hepatoma Cells. Cell Physiol Biochem. 2016;39: 1648-1662

## Claims

1. An isolated polypeptide comprising
a) the sequence of SEQ ID NO.1 or SEQ ID NO.2; or
b) a variant of any of the sequences of a) which is at least 80%, 81%, 82%, 83%, 84%, 85%, 86%, 87%, 88%, 89%, 90%, 91%, 92%, 93%, 94%, 95%, 96%, 97%, 98%, 99% identical SEQ ID NO.1 or SEQ ID NO.2, respectively,
for use in the prevention, relief and/or treatment of an overweight related or obesity related liver disorder or a pathological condition or disease caused by fatty liver, specifically non-alcoholic fatty liver disease (NAFLD) or non-alcoholic steatohepatitis (NASH) or metabolic associated fatty liver disease (MAFLD).

2. The polypeptide for use according to claim 1, wherein the isolated polypeptide further comprises at least one of the following amino acid substitutions based on the amino acid sequence of SEQ ID NO.1: H3C(Acm), F4R, R5Q, F7Nal1, S9K, A11S, a deletion of amino acids A11 to 116, or a combination thereof.

3. The polypeptide for use according to claims 1 or 2, wherein the isolated polypeptide comprises
a) the sequence of any of SEQ IN NO.3 or SEQ ID NO.4; or
b) a variant of any of the sequences of a) which is at least 80%, 81%, 82%, 83%, 84%, 85%, 86%, 87%, 88%, 89%, 90%, 91%, 92%, 93%, 94%, 95%, 96%, 97%, 98%, 99% identical to SEQ ID NO.3 or SEQ ID NO.4, respectively.

4. The polypeptide for use according to claims 1 to 3, wherein the pathological condition or disease is an overweight related or an obesity related liver disorder selected from steatosis, steatohepatitis, liver fibrosis or cirrhosis, non-alcoholic steatohepatitis (NASH), non-alcoholic fatty liver disease (NAFLD), metabolic associated fatty liver disease (MAFLD) and NAFLD related cirrhosis.

5. The polypeptide for use according to any one of claims 1 to 4, wherein the polypeptide consists of
i. the amino acid sequence of SEQ ID NO.1; or
ii. the amino acid sequence of SEQ ID NO.2; or
iii. the amino acid sequence of SEQ ID NO.3; or
iv. the amino acid sequence of SEQ ID NO.4.

6. The polypeptide for use according to any one of claims 1 to 5, consisting of
a) the murine SOCS1 protein (UniProt: 035716);
b) the human SOCS1 protein (UniProt: 015524); or
c) a variant of the sequence of a) or b) which is at least 80% homologous to the amino acid sequence of the murine SOCS1 protein or to the amino acid sequence of the human SOCS1 protein.

7. The polypeptide for use according to any one of claims 1 to 6, consisting of the sequence SEQ ID NO.1, SEQ ID NO.2, SEQ IN NO.3 or SEQ ID NO.4, wherein the polypeptide is bound to a cell permeability region.

8. A polypeptide for use according to any one of claims 1 to 7, wherein at least one amino acid of the sequence thereof is phosphorylated, preferably wherein at least one of the phosphorylated amino acids is a tyrosine (Y).

9. A composition, preferably a pharmaceutical composition, comprising a therapeutically effective amount of a polypeptide as defined in any one of claims 1 to 8, and at least one pharmaceutically acceptable vehicle or excipient, for use in the prevention, relief and/or treatment of an obesity related liver disorder or a pathological condition or disease caused by fatty liver, preferably wherein the pathological condition or disease caused by fatty liver is selected from steatosis, steatohepatitis, liver fibrosis or cirrhosis, more preferably non-alcoholic steatohepatitis (NASH), non-alcoholic fatty liver disease (NAFLD) and NAFLD related cirrhosis.

10. The composition for use according to claim 9, wherein the composition further comprises another active ingredient, preferably selected from vitamin E, semaglutide, tirzepatide, empaglifozin and losartan.

11. The composition for use according to claims 9 or 10, wherein the composition further comprises a pharmaceutically acceptable excipient.

12. The composition for use according to any one of claims 9 to 11, wherein said composition is suitable for oral, gastroenteric, parenteral, or rectal administration, preferably parenteral administration.
